# EUROPEAN PATENT APPLICATION

(11) **EP 3 560 950 A1**
(43) Date of publication of application: **30.10.2019**
(21) Application number: 17884946.9
(22) Date of filing: 20.12.2017
(51) Int. Cl.: C07K 7/08, C07K 19/00, C12N 15/12, C12N 15/63, A61K 38/10, A61P 25/30, A61P 25/04, A61P 25/16, A61P 25/28, A61P 25/18, A61P 25/24, A61P 35/00

(54) **NOVEL MUTANT OF ALFA-CONOTOXIN PEPTIDE TXID, PHARMACEUTICAL COMPOSITION AND USE THEREOF**

(30) Priority: 21.12.2016 CN 201611185856
(71) Applicant: Hainan University, Hainan 570228 (CN)
(72) Inventor: LUO, Sulan, Haikou Hainan 570228 (CN); ZHANGSUN, Dongting, Haikou Hainan 570228 (CN); ZHU, Xiaopeng, Haikou Hainan 570228 (CN); WU, Yong, Haikou Hainan 570228 (CN); YU, Jinpeng, Haikou Hainan 570228 (CN); MCINTOSH, J. Michael, Haikou Hainan 570228 (CN); CRAIK, David J., Haikou Hainan 570228 (CN)
(74) Representative: Kolster Oy Ab
(86) International application number: PCT/CN2017/117486
(87) International publication number: WO 2018/113697

(57) **Abstract**

The invention belongs to the field of biochemistry and molecular biology. The present invention provides a mutant of α-conotoxin peptide TxID, a pharmaceutical composition and use thereof. The TxID mutant is capable of specifically blocking acetylcholine receptors (nAChRs), in particular, α3β4, or α6/α3β4 subtype, and has application prospects in the manufacture of a medicament for smoking cessation and detoxification and analgesia, a medicament for treating psychiatric diseases and cancers, regulating appetite, and a tool medicine for neuroscience.

SEQUENCE LISTING

<110>  Hainan University

<120>  Novel Mutant of ¦Á-conotoxin Peptide TxID, Pharmaceutical Composition
and Use thereof

<130>  IEC170141PCT

<150>  201611185856.6
<151>  2016-12-21

<160>  44

<170>  PatentIn version 3.2

<210>  1
<211>  15
<212>  PRT
<213>  Artificial

<220>
<223>  TxID

<400>  1

Gly Cys Cys Ser His Pro Val Cys Ser Ala Met Ser Pro Ile Cys
1               5                   10                  15


<210>  2
<211>  15
<212>  PRT
<213>  Artificial

<220>
<223>  TxID[G1A]

<400>  2

Ala Cys Cys Ser His Pro Val Cys Ser Ala Met Ser Pro Ile Cys
1               5                   10                  15


<210>  3
<211>  15
<212>  PRT
<213>  Artificial

<220>
<223>  TxID[S4A]

<400>  3

Gly Cys Cys Ala His Pro Val Cys Ser Ala Met Ser Pro Ile Cys
1               5                   10                  15


<210>  4
<211>  15
<212>  PRT
<213>  Artificial

<220>
<223>  TxID[H5A]

<400>  4

Gly Cys Cys Ser Ala Pro Val Cys Ser Ala Met Ser Pro Ile Cys
1               5                   10                  15


<210>  5
<211>  15
<212>  PRT
<213>  Artificial

<220>
<223>  TxID[P6A]

<400>  5

Gly Cys Cys Ser His Ala Val Cys Ser Ala Met Ser Pro Ile Cys
1               5                   10                  15


<210>  6
<211>  15
<212>  PRT
<213>  Artificial

<220>
<223>  TxID[V7A]

<400>  6

Gly Cys Cys Ser His Pro Ala Cys Ser Ala Met Ser Pro Ile Cys
1               5                   10                  15


<210>  7
<211>  15
<212>  PRT
<213>  Artificial

<220>
<223>  TxID[S9A]

<400>  7

Gly Cys Cys Ser His Pro Val Cys Ala Ala Met Ser Pro Ile Cys
1               5                   10                  15


<210>  8
<211>  15
<212>  PRT
<213>  Artificial

<220>
<223>  TxID[M11A]

<400>  8

Gly Cys Cys Ser His Pro Val Cys Ser Ala Ala Ser Pro Ile Cys
1               5                   10                  15

<210>  9
<211>  15
<212>  PRT
<213>  Artificial

<220>
<223>  TxID[S12A]

<400>  9

Gly Cys Cys Ser His Pro Val Cys Ser Ala Met Ala Pro Ile Cys
1               5                   10                  15


<210>  10
<211>  15
<212>  PRT
<213>  Artificial

<220>
<223>  TxID[P13A]

<400>  10

Gly Cys Cys Ser His Pro Val Cys Ser Ala Met Ser Ala Ile Cys
1               5                   10                  15


<210>  11
<211>  15
<212>  PRT
<213>  Artificial

<220>
<223>  TxID[I14A]

<400>  11

Gly Cys Cys Ser His Pro Val Cys Ser Ala Met Ser Pro Ala Cys
1               5                   10                  15


<210>  12
<211>  15
<212>  PRT
<213>  Artificial

<220>
<223>  TxID[M11I]

<400>  12

Gly Cys Cys Ser His Pro Val Cys Ser Ala Ile Ser Pro Ile Cys
1               5                   10                  15


<210>  13
<211>  15
<212>  PRT
<213>  Artificial

<220>
<223>  TxID[S9A£¬M11L]

<400>  13

Gly Cys Cys Ser His Pro Val Cys Ala Ala Leu Ser Pro Ile Cys
1               5                   10                  15


<210>  14
<211>  15
<212>  PRT
<213>  Artificial

<220>
<223>  TxID[I14R]

<400>  14

Gly Cys Cys Ser His Pro Val Cys Ser Ala Met Ser Pro Arg Cys
1               5                   10                  15


<210>  15
<211>  15
<212>  PRT
<213>  Artificial

<220>
<223>  TxID[I14Y]

<400>  15

Gly Cys Cys Ser His Pro Val Cys Ser Ala Met Ser Pro Tyr Cys
1               5                   10                  15


<210>  16
<211>  15
<212>  PRT
<213>  Artificial

<220>
<223>  TxID[I14D]

<400>  16

Gly Cys Cys Ser His Pro Val Cys Ser Ala Met Ser Pro Asp Cys
1               5                   10                  15


<210>  17
<211>  15
<212>  PRT
<213>  Artificial

<220>
<223>  TxID-[S9Abu]


<220>
<221>  MISC_FEATURE
<222>  (9)..(9)

<223>  B is Abu

<400>  17

Gly Cys Cys Ser His Pro Val Cys Asx Ala Met Ser Pro Ile Cys
1               5                   10                  15


<210>  18
<211>  15
<212>  PRT
<213>  Artificial

<220>
<223>  TxID-[S9H]

<400>  18

Gly Cys Cys Ser His Pro Val Cys His Ala Met Ser Pro Ile Cys
1               5                   10                  15


<210>  19
<211>  15
<212>  PRT
<213>  Artificial

<220>
<223>  TxID-[S9R]

<400>  19

Gly Cys Cys Ser His Pro Val Cys Arg Ala Met Ser Pro Ile Cys
1               5                   10                  15


<210>  20
<211>  15
<212>  PRT
<213>  Artificial

<220>
<223>  TxID-[S9Y]

<400>  20

Gly Cys Cys Ser His Pro Val Cys Tyr Ala Met Ser Pro Ile Cys
1               5                   10                  15


<210>  21
<211>  15
<212>  PRT
<213>  Artificial

<220>
<223>  TxID-[S9T]

<400>  21

Gly Cys Cys Ser His Pro Val Cys Thr Ala Met Ser Pro Ile Cys
1               5                   10                  15

<210>  22
<211>  15
<212>  PRT
<213>  Artificial

<220>
<223>  TxID-[S12Y]

<400>  22

Gly Cys Cys Ser His Pro Val Cys Ser Ala Met Tyr Pro Ile Cys
1               5                   10                  15


<210>  23
<211>  15
<212>  PRT
<213>  Artificial

<220>
<223>  TxID-[I14L]

<400>  23

Gly Cys Cys Ser His Pro Val Cys Ser Ala Met Ser Pro Leu Cys
1               5                   10                  15


<210>  24
<211>  15
<212>  PRT
<213>  Artificial

<220>
<223>  TxID-[S9K]

<400>  24

Gly Cys Cys Ser His Pro Val Cys Lys Ala Met Ser Pro Ile Cys
1               5                   10                  15


<210>  25
<211>  15
<212>  PRT
<213>  Artificial

<220>
<223>  TxID-[S9L]

<400>  25

Gly Cys Cys Ser His Pro Val Cys Leu Ala Met Ser Pro Ile Cys
1               5                   10                  15


<210>  26
<211>  15
<212>  PRT
<213>  Artificial

<220>

<223>  TxID-[S9F]

<400>  26

Gly Cys Cys Ser His Pro Val Cys Phe Ala Met Ser Pro Ile Cys
1               5                   10                  15


<210>  27
<211>  16
<212>  PRT
<213>  Artificial

<220>
<223>  TxID-[14H]

<400>  27

Gly Cys Cys Ser His Pro Val Cys Ser Ala Met Ser Pro His Ile Cys
1               5                   10                  15


<210>  28
<211>  15
<212>  PRT
<213>  Artificial

<220>
<223>  TxID-[S9(D-Arg)]


<220>
<221>  MISC_FEATURE
<222>  (9)..(9)
<223>  X is D-Arg

<400>  28

Gly Cys Cys Ser His Pro Val Cys Xaa Ala Met Ser Pro Ile Cys
1               5                   10                  15


<210>  29
<211>  16
<212>  PRT
<213>  Artificial

<220>
<223>  TxID-[14D]

<400>  29

Gly Cys Cys Ser His Pro Val Cys Ser Ala Met Ser Pro Asp Ile Cys
1               5                   10                  15


<210>  30
<211>  15
<212>  PRT
<213>  Artificial

<220>
<223>  TxID-[S9(D-Ser)]

<220>
<221>  MISC_FEATURE
<222>  (9)..(9)
<223>  X is D-Ser

<400>  30

Gly Cys Cys Ser His Pro Val Cys Xaa Ala Met Ser Pro Ile Cys
1               5                   10                  15


<210>  31
<211>  15
<212>  PRT
<213>  Artificial

<220>
<223>  TxID-[S9E]

<400>  31

Gly Cys Cys Ser His Pro Val Cys Glu Ala Met Ser Pro Ile Cys
1               5                   10                  15


<210>  32
<211>  15
<212>  PRT
<213>  Artificial

<220>
<223>  TxID-[S9D]

<400>  32

Gly Cys Cys Ser His Pro Val Cys Asp Ala Met Ser Pro Ile Cys
1               5                   10                  15


<210>  33
<211>  15
<212>  PRT
<213>  Artificial

<220>
<223>  TxID-[M11H]

<400>  33

Gly Cys Cys Ser His Pro Val Cys Ser Ala His Ser Pro Ile Cys
1               5                   10                  15


<210>  34
<211>  15
<212>  PRT
<213>  Artificial

<220>
<223>  TxID-[H5W£¬S9A]

<400>  34

Gly Cys Cys Ser Trp Pro Val Cys Ala Ala Met Ser Pro Ile Cys
1               5                   10                  15


<210>  35
<211>  15
<212>  PRT
<213>  Artificial

<220>
<223>  TxID-[H5W]

<400>  35

Gly Cys Cys Ser Trp Pro Val Cys Ser Ala Met Ser Pro Ile Cys
1               5                   10                  15


<210>  36
<211>  16
<212>  PRT
<213>  Artificial

<220>
<223>  TxID-[9R]

<400>  36

Gly Cys Cys Ser His Pro Val Cys Arg Ser Ala Met Ser Pro Ile Cys
1               5                   10                  15


<210>  37
<211>  16
<212>  PRT
<213>  Artificial

<220>
<223>  TxID-[10R]

<400>  37

Gly Cys Cys Ser His Pro Val Cys Ser Arg Ala Met Ser Pro Ile Cys
1               5                   10                  15


<210>  38
<211>  13
<212>  PRT
<213>  Artificial

<220>
<223>  TP-2212-59

<400>  38

Gly Cys Cys Ser His Pro Asx Cys Phe Asx Glx Tyr Cys
1               5                   10


<210>  39

<211>  15
<212>  PRT
<213>  C. aulicus

<400>  39

Gly Cys Cys Ser Tyr Pro Pro Cys Phe Ala Thr Asn Pro Asp Cys
1               5                   10                  15


<210>  40
<211>  16
<212>  PRT
<213>  C. regius

<400>  40

Gly Cys Cys Ser His Pro Ala Cys Asn Val Asn Asn Pro His Ile Cys
1               5                   10                  15


<210>  41
<211>  16
<212>  PRT
<213>  Artificial

<220>
<223>  RegIIA[N11£¬12A]

<400>  41

Gly Cys Cys Ser His Pro Ala Cys Asn Val Ala Ala Pro His Ile Cys
1               5                   10                  15


<210>  42
<211>  16
<212>  PRT
<213>  C. pergrandis

<400>  42

Gly Cys Cys Ser His Pro Ala Cys Ser Val Asn His Pro Glu Leu Cys
1               5                   10                  15


<210>  43
<211>  18
<212>  PRT
<213>  C. purpurascens

<400>  43

Arg Asp Pro Cys Cys Ser Asn Pro Val Cys Thr Val His Asn Pro Gln
1               5                   10                  15


Ile Cys



<210>  44
<211>  13

<212>  PRT
<213>  C. bullatus

<400>  44

Gly Cys Cys Ser Thr Pro Pro Cys Ala Val Leu Tyr Cys
1               5                   10

## Description

### Technology Field

The invention pertains to the fields of biochemistry and molecular biology, and relates to a novel mutant of α-conotoxin peptide TxID, a pharmaceutical composition and use thereof.

### Background Art

Conotoxin (Conopeptide, CTx) is a neuropeptide secreted by tropical medicinal marine organisms, cone shells, which is used for predation and defense, and has specific functions of specifically binding various ion channels and receptors in animals (Terlau, H., and Olivera, B. M. (2004) Conus venoms: a rich source of novel ion channel-targeted peptides. Physiological reviews 84, 41-68. Schroeder, C. I., and Craik, D. J. (2012) Therapeutic potential of conopeptides. Future medicinal chemistry 4, 1243-1255).

Alpha-conotoxin is the currently found nicotinic acetylcholine receptor (nAChRs) subtype specific blocker with the best selectivity. Alpha-conotoxin and its target nAChRs are of great value in the study of various disease mechanisms and drug development. Alpha-conotoxin is one of the earliest discovered conotoxins, usually has small molecular weight, and generally is composed of 12-19 amino acid residues rich in disulfide bonds. There are many types of α-conotoxin with diverse activities and structures.

Nicotinic acetylcholine receptors (nAChRs) are cell membrane proteins with important physiological and clinical significance in the animal kingdom, are the receptors earliest discovered by humans, and can be divided into two types: muscular acetylcholine receptors and neuronal acetylcholine receptors. nAChRs are allosteric membrane proteins on cell membrane, which mediate many physiological functions of central and peripheral nervous systems, including learning, memory, response, analgesia, and motion control. nAChRs activate the release of various neurotransmitters such as dopamine, norepinephrine, serotonin, γ-aminobutyric acid (Gotti, C., and Clementi, F. (2004), Neuronal nicotinic receptors: from structure to pathology. Progress in neurobiology 74, 363-396). nAChRs have been shown to be key targets for screening drugs for treating a wide range of important diseases including pain, alcohol and drug addictions, mental retardation, dementia, schizophrenia, central nervous disorders, epilepsy, Parkinson's disease, mental diseases, neuromuscular block, myasthenia gravis, depression, hypertension, arrhythmia, asthma, muscle relaxation, stroke, breast cancer and lung cancer (Gotti, C., Moretti, M., Bohr, I., Ziabreva , I., Vailati, S., Longhi, R., Riganti, L., Gaimarri, A., McKeith, I.G., Perry, R.H., Aarsland, D., Larsen, J.P., Sher, E., Beattie, R., Clementi, F., and Court, JA (2006) Selective nicotinic acetylcholine receptor subunit deficits identified in Alzheimer's disease, Parkinson's disease and dementia with Lewy bodies by immunoprecipitation. Neurobiology of disease 23, 481-489. Lee, C.H., Huang, C.S., Chen, C.S., Tu, S.H., Wang, Y.J., Chang, Y.J., Tam, K.W., Wei, P.L., Cheng, T.C., Chu, J.S., Chen, L.C., Wu, C.H., and Ho, Y.S. (2010) Overexpression and Activation of t He alpha9-nicotinic receptor during tumorigenesis in human breast epithelial cells. Journal of the National Cancer Institute 102, 1322-1335. Lee, C.H., Chang, Y.C., Chen, C.S., Tu, S.H., Wang, Y.J., Chen, L.C., Chang , Y.J., Wei, P.L., Chang, H.W., Chang, C.H., Huang, C.S., Wu, C.H., and Ho, Y.S. (2011) Crosstalk between nicotine and estrogen-induced estrogen receptor activation induces alpha 9-nicotinic acetylcholine receptor expression in human breast cancer cells. Breast Cancer Res Tr 129, 331-345. Rahman, S. (2013) Nicotinic Receptors as Therapeutic Targets for Drug Addictive Disorders. Cns Neurol Disord-Dr 12, 633-640). nAChRs are assembled into a wide variety of subtypes from different alpha and beta subunits, each with distinct pharmacological characteristics. The development of drugs as ligands with high selectivity for various subtypes of nAChRs is the key to treat these diseases (Livett BG, Sandall DW, Keays D, Down J, Gayler KR, Satkunanathan N, Khalil Z. Therapeutic applications of conotoxins that target the neuronal nicotinic acetylcholine receptor. Toxicon, 2006, 48(7): 810-829; Taly A, Corringer PJ, Guedin D, Lestage P, Changeux JP. Nicotinic receptors: allosteric transitions and therapeutic targets in the nervous system. Nat Rev Drug Discov. 2009, 8(9): 733-50; Layla A, McIntosh JM. Alpha-conotoxins as pharmacological probes of nicotinic acetylcholine receptors [J]. Acta Pharmacol Sin 2009 Jun; 30 (6): 771-783). However, the premise for the development of such drugs is to obtain selective compounds that can specifically bind to various subtypes of nAChRs, can be used as tools to study and identify the fine composition and physiological functions of various subtypes, or can be directly used as medicaments for treatment of the related diseases.

Drug addiction is both a medical problem and a serious social problem. Cigarette addiction is caused by nicotine in tobacco, and its receptors in body are nicotinic acetylcholine receptors (nAChRs) (Azam L, McIntosh JM. Alpha-conotoxins as pharmacological probes of nicotinic acetylcholine receptors. Acta Pharmacol Sin. 2009; 30(6): 771-783). Studies have shown that blocking nAChRs containing α3β4 is effective in preventing the onset of craving for tobacco, morphine and cocaine addiction, and significantly inhibits the desire to smoke and take drugs (Brunzell DH, Boschen KE, Hendrick ES, Beardsley PM, McIntosh JM. Alpha-conotoxin Mil-sensitive nicotinic acetylcholine receptors in the nucleus accumbens shell regulate progressive ratio responding maintained by nicotine, Neuropsychopharmacology, 2010, 35(3):665-73).

α3β4 nAChR is the major acetylcholine receptor subtype in the sensory and autonomous nerve centers. α3β4 nAChRs are also branches of central nervous system (CNS) neurons, such as the centrally extending habenula and dorsal bone marrow, which are involved in the addiction of nicotine and other abused drugs (Millar, N. S.; Gotti, C., Diversity of vertebrate nicotinic acetylcholine receptors. Neuropharmacology 2009,56 (1), 237-46; Tapper, A. R.; McKinney, S. L.; Nashmi, R.; Schwarz, J.; Deshpande, P.; Labarca, C.; Whiteaker, P.; Marks, M. J.; Collins, A. C.; Lester, H. A., Nicotine activation of alpha4* receptors: sufficient for reward, tolerance, and sensitization. Science 2004,306 (5698), 1029-32). α3β4 nAChR is involved in the central limb dopamine pathway and plays a very important role in the reward effect of the abuse of certain substances (such as nicotine, drugs, etc). (Jackson KJ, Sanjakdar SS, Muldoon PP, McIntosh JM, Damaj MI. The α3β4* nicotinic acetylcholine receptor subtype mediates nicotine reward and physical nicotine withdrawal signs independently of the a5 subunit in the mouse. Neuropharmacology. 2013 Jul; 70:228-35). α3β4 nAChR blockers can effectively curb cocaine addiction, or cocaine and nicotine co-produced drug addiction (Khroyan TV, Yasuda D, Toll L, Polgar WE, Zaveri NT. High affinity α3β4 nicotinic acetylcholine receptor ligands AT-1001 and AT-1012 attenuate cocaineinduced conditioned place preference and behavioral sensitization in mice. Biochem Pharmacol. 2015 Oct 15; 97(4): 531-41). In addition, nicotine-induced exercise and reward effects were significantly reduced in beta-4 subunit knockout mice, which means that α3β4 nAChR plays an important role in nicotine addiction in CNS. The experimental results show that α3β4 nAChR blocker can also effectively prevent the onset of craving and alcohol addiction. (Toll L, Zaveri NT, Polgar WE, Jiang F, Khroyan TV, Zhou W, Xie XS, Stauber GB, Costello MR, Leslie FM. AT-1001: a high affinity and selective α3β4 nicotinic acetylcholine receptor antagonist blocks nicotine self-administration in rats. Neuropsychopharmacology. 2012 May; 37(6):1367-76. Cippitelli A, Wu J, Gaiolini KA, Mercatelli D, Schoch J, Gorman M, Ramirez A, Ciccocioppo R, Khroyan TV, Yasuda D, Zaveri NT, Pascual C, Xie XS, Toll L. AT-1001: a high-affinity α3β4 nAChR ligand with novel nicotine-suppressive pharmacology. Br J Pharmacol. 2015 Apr;172(7):1834-45. Slimak MA, Ables JL, Frahm S, Antolin-Fontes B, Santos-Torres J, Moretti M, Gotti C, Ibañez-Tallon I. Habenular expression of rare missense variants of the β4 nicotinic receptor subunit alters nicotine consumption. Front Hum Neurosci. 2014 Jan 27; 8:12. Cippitelli A, Brunori G, Gaiolini KA, Zaveri NT, Toll L. Pharmacological stress is required for the anti-alcohol effect of the α3β4* nAChR partial agonist AT-1001. Neuropharmacology. 2015 Jun; 93:229-36. Rahman S, Prendergast MA. Cholinergic receptor system as a target for treating alcohol abuse and dependence. Recent Pat CNS Drug Discov. 2012 Aug;7(2):145-50).

α3β4 nAChR also plays an important role in the fear response, which is essential for regulating the release of glutamate and norepinephrine (Zhu, P. J.; Stewart, R. R.; McIntosh, J. M.; Weight, F. F., Activation of nicotinic acetylcholine receptors increases the frequency of spontaneous GABAergic IPSCs in rat basolateral amygdala neurons. Journal of neurophysiology 2005, 94 (5), 3081-91. Alkondon, M.; Albuquerque, E. X., A non-alpha7 nicotinic acetylcholine receptor modulates excitatory input to hippocampal CA1 interneurons. Journal of neurophysiology 2002,87 (3), 1651-4. Luo, S.; Kulak, J. M.; Cartier, G. E.; Jacobsen, R. B.; Yoshikami, D.; Olivera, B. M.; McIntosh, J. M., alpha-conotoxin AuIB selectively blocks alpha3 beta4 nicotinic acetylcholine receptors and nicotine-evoked norepinephrine release. The Journal of neuroscience : the official journal of the Society for Neuroscience 1998,18 (21), 8571-9. Kulak, J. M.; McIntosh, J. M.; Yoshikami, D.; Olivera, B. M., Nicotine-evoked transmitter release from synaptosomes: functional association of specific presynaptic acetylcholine receptors and voltage-gated calcium channels. Journal of neurochemistry 2001,77 (6), 1581-9). By using α-conotoxin, α3β4 nAChR is identified to be enriched and expressed in human adrenal pheochromocytoma. (Hone AJ, McIntosh JM, Azam L, Lindstrom J, Lucero L, Whiteaker P, Passas J, Blázquez J, Albillos A. α-Conotoxins Identify the α3β4* Subtype as the Predominant Nicotinic Acetylcholine Receptor Expressed in Human Adrenal Chromaffin Cells. Mol Pharmacol. 2015 Nov; 88(5):881-93). α3β4 nAChR plays an important role in the olfactory nerve, which mediates the screening and filtering of the mitral cell response, and stimulates the excitation of nerve cells by activating α3β4 nAChR on the mitral cells (D'Souza RD, Vijayaraghavan S. Nicotinic receptor-mediated filtering of mitral cell responses to olfactory nerve inputs involves the α3β4 subtype. J Neurosci. 2012 Feb 29; 32(9): 3261-6).

nAChRs containing α3-subunits, including α3β2 and α3β4 subtypes, are mainly expressed in the peripheral nervous system and are targets of neuralgia drugs. Alpha-conotoxin, which blocks α3β2 or α3β4 nAChRs, shows excellent analgesic activity in a variety of preclinical chronic pain models and is not addictive. Intractable pain is a worldwide health problem, and new therapeutic drugs are urgently needed (Napier, I. A.; Klimis, H.; Rycroft, B. K.; Jin, A. H.; Alewood, P. F.; Motin, L.; Adams, D. J.; Christie, M. J., Intrathecal α-conotoxins Vc1.1, AuIB and Mil acting on distinct nicotinic receptor subtypes reverse signs of neuropathic pain. Neuropharmacology 2012, 62 (7), 2202-2207. Blyth, F. M.; March, L. M.; Brnabic, A. J.; Jorm, L. R.; Williamson, M.; Cousins, M. J., Chronic pain in Australia: a prevalence study. PAIN 2001, 89 (2-3), 127-34. Cousins, M. J.; Brennan, F.; Carr, D. B., Pain relief: a universal human right. PAIN 2004,112 (1-2), 1-4. Eisenberg, E.; McNicol, E. D.; Carr, D. B., Efficacy and safety of opioid agonists in the treatment of neuropathic pain of nonmalignant origin: systematic review and meta-analysis of randomized controlled trials. JAMA: the journal of the American Medical Association 2005,293 (24), 3043-52). According to a survey, pain affects almost 1 in 6 people, including arthritis, neuralgia, swelling and pain. Neuropathic pains affect 4-8% of people, and neuralgia may be induced by alcoholism, sciatica, cancer and cancer chemotherapy, diabetes, trigeminal neuralgia, sclerosis, herpes zoster, mechanical injuries and surgical injuries, etc.

The function of α6/α3β4 (α6β4*, * indicates other possible subunits) nAChR subtype is also very important. This subtype is widely distributed in important parts such as human adrenal chromaffin cells, rat dorsal root ganglion (DRG) neurons, adolescence rat hippocampus, noradrenergic nerve endings. α6β4* nAChRs are dominant in human adrenal chromaffin cells, whereas α3β4 nAChRs are dominant in rodent adrenal chromaffin cells (Hernandez-Vivanco A, Hone AJ, Scadden ML, Carmona-Hidalgo B, McIntosh JM, Albillos A. Monkey adrenal chromaffin cells express α6β4* nicotinic acetylcholine receptors. PLoS One. 2014 Apr 11; 9(4):e94142). Acetylcholine receptors present in the dorsal root ganglia (DRG) are potential targets for analgesic drugs. Studies have shown that there are indeed neurons in DRG that primarily express α6β4* and α3β4 nAChRs (Hone AJ, Meyer EL, McIntyre M., McIntosh JM. Nicotinic acetylcholine receptors in dorsal root ganglion neurons include the α6β4* subtype. FASEB J. 2012 Feb; 26(2): 917-26. Smith NJ, Hone AJ, Memon T, Bossi S, Smith TE, McIntosh JM , Olivera BM, Teichert RW. Comparative functional expression of nAChR subtypes in rodent DRG neurons. Front Cell Neurosci. 2013 Nov 28; 7:225). Therefore, α6β4* and α3β4 nAChRs in the peripheral nervous system DRG are potential targets for neuralgia drugs, and the blockers for these two subtypes are expected to be developed as novel analgesics.

Interestingly, α3β4 nAChR mediates the growth of small cell lung cancer (SCLC) cells, and signal transduction through this receptor promotes the development of lung cancer. α3β4 nAChR blocker α-conotoxin AuIB is effective in inhibition of variability and growth of SCLC cells, which means that antagonists that specifically block α3β4 nAChR are expected to be developed as novel therapeutic agents for the treatment of small cell lung cancer (Improgo MR, Soll LG, Tapper AR, Gardner PD. Nicotinic acetylcholine receptors mediate lung Cancer growth. Front Physiol. 2013 Sep 17; 4:251). In addition, studies have shown that specifically blocking α3β4 or α6/α3β4 nAChRs on tumor cells of leukemia, lung cancer, and neuroblastoma is expected to achieve anticancer effects (Improgo MR, Soll LG, Tapper AR, Gardner PD. Nicotinic acetylcholine receptors mediate lung cancer growth. Front Physiol. 2013 Sep 17; 4:251).

In addition, some studies have shown that activation of hypothalamic α3β4 nAChR activates pro-opiomelanocortin (POMC) neurons in mouse experimental models; POMC neurons and subsequent activation of melanocortin 4 receptors play a key role in nicotine-induced loss of appetite, i.e., regulating the loss of appetite (anorexia) or the enhancement of appetite through specific agonists or specific blockers of α3β4 nAChR; nAChR receptors containing β4 subunits, such as α3β4 or α6/α3β4 nAChRs etc., play a key role in the regulation of appetite in mammals; the specific agonist of α3β4 or α6/α3β4 nAChRs has the potential to develop diet drugs; the specific antagonist of α3β4 or α6/α3β4 nAChRs has the potential to develop drugs for the treatment of anorexia, or to screen for diet drugs (Yann S. Mineur, Alfonso Abizaid, Yan Rao, Ramiro Salas, Ralph J. DiLeone, Daniela Gündisch, Sabrina Diano, Mariella De Biasi, Tamas L Horvath, Xiao-Bing Gao, Marina R. Picciotto. Nicotine Decreases Food Intake through Activation of POMC Neurons. Science, 2011, 332, 1330-1332). Therefore, a specific blocker of α3β4 or α6/α3β4 nAChRs is expected to be used for screening weight-loss drugs, as well as for the development of new drugs for the treatment of appetite and body weight-related metabolic diseases.

At present, there is an urgent need to develop new highly specific blockers targeting different subtypes of nAChRs, particularly specific blockers for α3β4 or α6/α3β4 nAChRs.

### Contents of the Invention

The inventors have intensively studied and creatively worked to discover a series of new mutants of the α-conotoxin peptide TxID, which are capable of specifically blocking acetylcholine receptors, particularly those having selectivity and strong blocking activity on α3β4 nAChR which is a drug target of addiction, pain, and small cell lung cancer, and those having strong blocking activity on pain drug target α6β4* (* indicates other possible subunits) nAChRs, and has excellent application prospects in preparing drugs for smoking cessation, detoxification and analgesia, developing therapeutic drugs for small cell lung cancer, depression, dementia, schizophrenia, Parkinson's disease, and developing tool drugs for neurosciences. The following invention is thus provided:
One aspect of the invention relates to an isolated polypeptide having an amino acid sequence as shown in SEQ ID NO: 1 in which the serine at position 9 is substituted by a different L-form or D-form amino acid; preferably, the serine at position 9 in the sequence shown in SEQ ID NO: 1 is replaced by alanine, 2-aminobutyric acid, histidine, arginine, tyrosine, threonine, lysine, leucine, phenylalanine, D-arginine, D-serine, glutamic acid or aspartic acid. The different L-form or D-form amino acid means that the amino acid is different from the serine at position 9, that is, the amino acid at position 9 after the substitution is not L-serine.

The present invention also relates to an isolated polypeptide having an amino acid sequence as shown in SEQ ID NO: 1 in which an L-form or a D-form amino acid is inserted between the serine at position 9 and the 10th position, and the L-form or D-form amino acid is not L-serine; preferably, an alanine, 2-aminobutyric acid, histidine, arginine, tyrosine, threonine, lysine, leucine, phenylalanine, D-arginine, D-serine, glutamic acid or aspartic acid is inserted between the 9th and 10th positions in the sequence shown in SEQ ID NO: 1.

One aspect of the invention relates to an isolated polypeptide having an amino acid sequence as set forth in any one of SEQ ID NOs: 2-3, 7, 9, 11-33, respectively.

In one embodiment of the invention, the polypeptide, wherein counted from the N-terminus of the polypeptide:
the first cysteine forms a disulfide bond with the third cysteine, and the second cysteine forms a disulfide bond with the fourth cysteine; or the first cysteine forms a disulfide bond with the fourth cysteine, and the second cysteine forms a disulfide bond with the third cysteine; or the first cysteine forms a disulfide bond with the second cysteine, and the third cysteine forms a disulfide bond with the fourth cysteine.

In one embodiment of the invention, the carboxy terminus of the polypeptide is amidated.

Although the wild-type α-conotoxin TxID has been disclosed in the inventors' previous patents and articles, many studies have shown that for conotoxin peptides, even one different amino acid in sequence may result in new receptor specificity, which means a new tool is provided in the study of receptors, and a new drug candidate and lead drug is provided in the development of new drugs (Conotoxin peptide, Lu Bosong, Huang Peitang, 1999, patent, application number: 99106070.9. Halai, R ., Clark, R.J., Nevin, S.T., Jensen, J.E., Adams, D.J., and Craik, D.J. (2009). Scanning mutagenesis of alpha-conotoxin Vc1.1 reveals residues crucial for activity at the alpha9alpha10 nicotinic acetylcholine receptor. The Journal of Biological Chemistry 284, 20275-20284. Kompella SN, Hung A, Clark RJ, Mari F, Adams D.J., 2015. Alanine scan of alpha-conotoxin RegIIA reveals a selective alpha3beta4 nicotinic acetylcholine receptor antagonist. J Biol Chem 290: 1039-1048). The findings of the present invention (Table 2, Table 3) further confirm this fact.

The present invention identifies key amino acids that interact between the TxID mutant and the two subtypes α3β4 and α6/α3β4 nAChR (see Table 2), except for the maintenance of disulfide-linked cysteines (Cys, C), including histidine at position 5 (TxID[H5A]), proline at position 6 (TxID[P6A]), valine at position 7 (TxID[V7A]), methionine at position 11 (TxID[M11A]), proline at position 13 (TxID[P13A]), these 5 amino acids on TxID play a crucial role in interaction with α3β4 and α6/α3β4 nAChR receptors, no matter which one amino acid is replaced by alanine (Ala, A), their blocking activities on both subtypes are completely lost, their half-blocking doses (IC₅₀) are all above 10000 nM, that is, at a high concentration of 10 µM, these mutants have current blockades of not more than 50% on two subtypes α3β4 and α6/α3β4 nAChR, or no blockade at all. See Table 2 for details.

In addition, the serine (Ser, S) at position 9 on TxID is also important for the binding of the subtype α6/α3β4 nAChR. If the serine at this position is replaced with alanine, arginine, or lysine (TxID[S9A], TxID[S9R], TxID[S9K]), it would result in a significant decrease in the blocking activity with α6/α3β4 nAChR, for example, the blocking activities of TxID[S9A] and TxID[S9R] to α6/α3β4 nAChR are decreased by 5.2 and 7.8 times respectively in comparison with the wild type TxID, while that of TxID[S9K] is decreased by 534.8 times. This illustrates the importance of the serine at position 9 on TxID for the binding of the subtype α6/α3β4 nAChR. However, if the serine at position 9 on TxID is mutated into 2-aminobutyric acid (TxID[S9Abu]), histidine (TxID[S9H]), tyrosine (TxID[S9Y]), threonine (TxID[S9T]), leucine (TxID[S9L]), phenylalanine (TxID[S9F]), respectively, the resultant mutants substantially maintain the binding activities to α3β4 and α6/α3β4 nAChR receptors, which are not significantly different from the activity of the wild type TxID. If the serine at position 9 on TxID is mutated into D-arginine (TxID[S9(D-Arg)]), D-serine (TxID[S9(D-Ser)]), glutamic acid (TxID[S9E]), aspartic acid TxID[S9D], respectively, the binding activities of these resultant mutants to α3β4 and α6/α3β4 nAChR receptors are decreased significantly by at least 10 times, with a decreasing amplitude between 10 and 303 times (Table 2). The binding activity of TxID[S9E] to α3β4 nAChR receptor is abruptly attenuated by more than 100 times, and the binding (blocking) activity to α6/α3β4 nAChR receptor is completely lost (Table 2). Thus, the serine at position 9 on TxID is also important for the binding activity of receptor, and the activity of mutant changes depending on the type of amino acid that replaces the position.

The glycine at position 1 on TxID is also important for the binding activity of receptor. The activity of the mutant with alanine substituted for the glycine (TxID[G1A]) is significantly decreased, and its binding activities to α3β4 and α6/α3β4 nAChR receptors are decreased by 17 times and 8.2 times respectively. The results of the present invention confirm the high diversity of the amino acid sequence of conotoxin and its biologically active function, and even one different amino acid in the conotoxin sequence may result in a functional change.

In addition, the inventors found 5 new TxID mutants: SEQ ID NO: 7 that is TxID[S9A], SEQ ID NO: 19 that is TxID[S9R], SEQ ID NO: 24 that is TxID[S9K], SEQ ID NO: 29 that is TxID[14D] and SEQ ID NO: 31 that is TxID[S9E], which have a good discrimination on two subtypes α3β4 and α6β4* nAChRs with a discrimination range of more than 45 times, and even can completely discriminate them, that is, they have blocking activity on α3β4 nAChRs, but almost no blocking activity on α6β4* nAChRs (Table 3). Among them, TxID[S9E] shows good selectivity to α3β4 nAChR receptor, while its blocking activity on α3β4 nAChR receptor is weak, but it completely loses the binding activity to α6/α3β4 nAChR receptor. This unique advantage makes the above-mentioned polypeptides as tools, which have important scientific significance and high application value in distinguishing similar subtypes such as α3β4 and α6/α3β4 nAChR. All these results show that they have extremely high application value for the design and development of molecular probes in the structural and functional studies of α3β4 and α6/α3β4 nAChR subtypes, as well as for new drug development.

In the present invention, a novel α-conotoxin (named as a new mutant of α-CTx TxID) is obtained, which is a strong blocker for α3β4 nAChR, and is also the most active α3β4 nAChR blocker discovered so far, and also has strong or certain blocking effects on α6/α3β4 nAChR, but has no or extremely weak blocking activity on other nAChR subtypes. There are very few ligand materials that can effectively distinguish two similar subtypes, α3β4 and α6β4* nAChRs. Thus, the new mutant of α-conotoxin TxID series is a novel tool for the study of the structure and function of α3β4 or α6β4* nAChRs, and contributes to the development of therapeutic drugs for addiction, pain, cancer, fear and the like.

In one embodiment of the invention, the polypeptide is for use in the treatment and/or prevention of a nervous system disease or cancer, or for use in killing pests, analgesia, smoking cessation, detoxification or promoting appetite;
preferably, the nervous system disease is at least one of addiction, neuralgia, Parkinson's disease, dementia, schizophrenia and depression;
preferably, the addiction is caused by at least one of the following factors: various psychoactive substances such as nicotine, opium, heroin, methamphetamine (ice), morphine, marijuana, cocaine or alcohol;
preferably, the neuralgia is selected from at least one of the following: sciatica, trigeminal neuralgia, lymphatic neuralgia, multi-point motor neuralgia, acute strenuous spontaneous neuralgia, crush neuralgia, and compound neuralgia;
preferably, the neuralgia is caused by at least one of the following factors: cancer, cancer chemotherapy, alcoholism, diabetes, sclerosis, herpes zoster, mechanical injury, surgical injury, AIDS, head neuralgia, drug poisoning, industrial pollution poisoning, myeloma, chronic congenital sensory neuropathy, angiitis, vasculitis, ischemia, uremia, childhood bile liver disease, chronic respiratory disorder, multiple organ failure, sepsis/pyaemia, hepatitis, porphyria, vitamin deficiency, chronic liver disease, native biliary sclerosis, hyperlipidemia, leprosy, Lyme arthritis, sensory perineuritis or allergies;
preferably, the cancer is a lung cancer such as small cell lung cancer, ovarian cancer or breast cancer.

The above polypeptide may be a chemically synthesized amino acid sequence (for example, by referring to the method in Example 1); or a polypeptide obtained by expressing a nucleotide via means of genetic recombination (the preparation of the nucleotide sequence may refer to a conventional method of molecular biology); also obtained by referring to the following method:
The method for preparing the polypeptide of the present invention comprises the following steps:
1) a linear polypeptide is synthesized by peptide synthesizer or manual method, wherein the side chain protecting groups of Fmoc amino acids are: Pmc (Arg), But (Thr, Ser, Tyr), OBut (Asp), Boc (Lys); the protecting group for cysteine is Trt or Acm;
2) the linear polypeptide obtained in the step 1) is cleaved from the resin, and the crude linear polypeptide is recovered by precipitation and washing with ice diethyl ether, and preparative reverse-HPLC C18 column (Vydac) is used for purification;
3) the product obtained in step 2) is subjected to two-step oxidative folding.

A further aspect of the invention relates to an isolated fusion protein comprising at least one polypeptide of the invention.

The present invention also encompasses a fusion polypeptide or a cleavable fusion polypeptide in which an additional peptide/polypeptide is fused at the N-terminus and/or C-terminus of α-conotoxin peptide of the present invention. The techniques for producing fusion polypeptides are known in the art and include ligating a coding sequence encoding a peptide of the invention with a coding sequence encoding the additional peptide/polypeptide such that they are in the same reading frame and the expression of the fusion polypeptide is controlled by the same promoter and terminator.

A further aspect of the invention relates to an isolated polynucleotide encoding a polypeptide of the invention.

A further aspect of the invention relates to a nucleic acid construct comprising a polynucleotide of the invention; preferably, the nucleic acid construct is a recombinant vector; preferably, the nucleic acid construct is a recombinant expression vector.

A further aspect of the invention relates to a transformed cell comprising a polynucleotide of the invention, or a nucleic acid construct of the invention.

A further aspect of the invention relates to a pharmaceutical composition comprising at least one polypeptide of the invention; optionally, further comprising a pharmaceutically acceptable excipient.

The pharmaceutical composition can be used to study, diagnose, ameliorate or treat diseases or conditions associated with addiction, neuralgia, cancer, mental retardation, pain, Parkinson's disease, psychosis, depression, myasthenia gravis, and the like. In one embodiment, a pharmaceutical composition comprising a therapeutically effective amount of a peptide of the invention is formulated and administered in a pharmaceutically acceptable manner, taking into account the clinical condition, delivery site, method of administration, schedule of administration of an individual patient and other factors known to a doctor. The term "effective amount" herein used is therefore determined by these considerations.

A pharmaceutical composition comprising a therapeutically effective amount of a polypeptide of the present invention is administered parenterally, orally, intracisternally, intrathecally, and the like. The term "pharmaceutically acceptable carrier" means a non-toxic solid, semi-solid or liquid filler, diluent, capsule material or any type of formulation excipient. The term "parenteral" as used herein refers to a mode of administration including intravenous, intramuscular, intraperitoneal, intrasternal, subcutaneous, intrathecal, and intraarticular injections and infusions. The polypeptide of the present invention can also be administered properly by a sustained release system.

A further aspect of the invention relates to a use of a polypeptide of the invention in the manufacture of a medicament for blocking an acetylcholine receptor; preferably, the acetylcholine receptor is an α3β4 acetylcholine receptor, or an α6β4* acetylcholine receptor such as α6/α3β4 acetylcholine receptor.

A further aspect of the invention relates to a use of a polypeptide or pharmaceutical composition of the invention in the manufacture of a medicament for the treatment and/or prevention of a nervous system disease or cancer, or in the manufacture of a medicament for killing pests, analgesia, smoking cessation, detoxification or promoting appetite;
preferably, the nervous system disease is at least one of addiction, neuralgia, Parkinson's disease, dementia, schizophrenia and depression;
preferably, the addiction is caused by at least one of the following factors: various psychoactive substances such as nicotine, opium, heroin, methamphetamine (ice), morphine, marijuana, cocaine or alcohol;
preferably, the neuralgia is selected from at least one of the following: sciatica, trigeminal neuralgia, lymphatic neuralgia, multi-point motor neuralgia, acute strenuous spontaneous neuralgia, crush neuralgia, and compound neuralgia;
preferably, the neuralgia is caused by at least one of the following factors: cancer, cancer chemotherapy, alcoholism, diabetes, sclerosis, herpes zoster, mechanical injury, surgical injury, AIDS, head neuralgia, drug poisoning, industrial pollution poisoning, myeloma, chronic congenital sensory neuropathy, angiitis, vasculitis, ischemia, uremia, childhood bile liver disease, chronic respiratory disorder, multiple organ failure, sepsis/pyaemia, hepatitis, porphyria, vitamin deficiency, chronic liver disease, native biliary sclerosis, hyperlipidemia, leprosy, Lyme arthritis, sensory perineuritis or allergies;
preferably, the cancer is a lung cancer such as small cell lung cancer, ovarian cancer, leukemia, neuroblastoma or breast cancer.

Prior art studies have shown that α3β4 nAChR is a drug target for the treatment of neuropsychiatric diseases such as addictions caused by nicotine, morphine and cocaine, neuralgia, small cell lung cancer, Parkinson's disease, dementia, schizophrenia, depression, fear, etc. In addition, α6β4* nAChRs is also a potential target for many diseases, and is also an action target for pain drugs (see related references in the background art). Therefore, the novel α-conotoxin TxID mutant of the present invention has extremely high application value in the mechanism research, diagnosis and treatment of the above diseases.

A further aspect of the invention relates to a method of blocking an acetylcholine receptor or modulating the level of acetylcholine in vivo or in vitro, comprising the step of administering to a subject or administering to a cell an effective amount of a polypeptide or pharmaceutical composition of the invention; preferably, the acetylcholine receptor is an α3β4 acetylcholine receptor or an α6β4* acetylcholine receptor such as α6/α3β4 acetylcholine receptor.

A further aspect of the invention relates to a method of treating and/or preventing a nervous system disease or cancer, or a method of killing pests, analgesia, smoking cessation, detoxification or promoting appetite, comprising a step of administering to a subject an effective amount of a polypeptide or pharmaceutical composition of the invention;
preferably, the nervous system disease is at least one of addiction, neuralgia, Parkinson's disease, dementia, schizophrenia and depression;
preferably, the addiction is caused by at least one of the following factors: various psychoactive substances such as nicotine, opium, heroin, methamphetamine (ice), morphine, marijuana, cocaine or alcohol;
preferably, the neuralgia is selected from at least one of the following: sciatica, trigeminal neuralgia, lymphatic neuralgia, multi-point motor neuralgia, acute strenuous spontaneous neuralgia, crush neuralgia, and compound neuralgia;
preferably, the neuralgia is caused by at least one of the following factors: cancer, cancer chemotherapy, alcoholism, diabetes, sclerosis, herpes zoster, mechanical injury, surgical injury, AIDS, head neuralgia, drug poisoning, Industrial pollution poisoning, myeloma, chronic congenital sensory neuropathy, angiitis, vasculitis, ischemia, uremia, childhood bile liver disease, chronic respiratory disorder, multiple organ failure, sepsis/pyaemia, hepatitis, Porphyria, vitamin deficiency, chronic liver disease, native biliary sclerosis, hyperlipidemia, leprosy, Lyme arthritis, sensory perineuritis or allergies;
preferably, the cancer is a lung cancer such as small cell lung cancer, ovarian cancer, leukemia, neurocytoma or breast cancer.

The administration dosage depends on a number of factors, such as the severity of the condition to be treated, the sex, age, weight and individual response of a patient or animal, as well as the condition and prior medical history of the patient to be treated. It is common practice in the art to start with a dose that is lower than that required to achieve the desired therapeutic effect, gradually increasing the dosage until the desired effect is achieved.

In the present invention, the term "addiction" means that a subject who repeatedly uses a psychoactive substance is in a state of periodic or chronic poisoning. The psychoactive substance refers to nicotine, opium, heroin, methamphetamine (ice), morphine, marijuana, cocaine, and other narcotic drugs and psychotropic substances that are officially regulated and can cause addiction. Addiction is associated with a large amount of dopamine produced in brain. It is manifested by the uncontrollable application of preferred substances and the difficulty of self-control or the difficult to correct application behavior. For the purpose of obtaining psychoactive substances to achieve good feelings or avoid withdrawal pain, the subject can be unscrupulous. Typically, tolerance is increased and withdrawal symptoms often occur after discontinuation of using substance. The life of an addict may be dominated by material use, thus seriously affecting and even abandoning other important activities and all responsibilities. Therefore, the use of substances both harms individuals and society. When used in alcohol, it is equivalent to the concept of chronic alcoholism. The term addiction also covers both physical and psychological aspects. Psychological addiction emphasizes the experience of impaired self-control of drinking and taking drugs, while physical addiction refers to tolerance and withdrawal symptoms.

The term "nucleic acid construct", as defined herein, is a single- or double-stranded nucleic acid molecule, preferably an artificially constructed nucleic acid molecule. Optionally, the nucleic acid construct further comprises one or more operably linked regulatory sequences.

In the present invention, the term "operably linked" refers to a spatial arrangement of the functionality of two or more nucleotide regions or nucleic acid sequences. The "operably linked" can be achieved by means of genetic recombination.

In the present invention, the term "vector" refers to a nucleic acid delivery vehicle into which a polynucleotide inhibiting a certain protein can be inserted. For example, the vectors include: plasmids; phagemids; cosmids; artificial chromosomes such as yeast artificial chromosomes (YAC), bacterial artificial chromosomes (BAC) or P1 derived artificial chromosomes (PAC); phages such as lambda phage or M13 phage; and animal viruses, etc. The animal viruses used as vectors include retroviruses (including lentiviruses), adenoviruses, adeno-associated viruses, herpes viruses (such as herpes simplex virus), poxviruses, baculoviruses, papillomaviruses, papovavirus (such as SV40). A vector may contain a variety of elements that control expression.

In the present invention, the term "host cell" refers to a cell into which a vector is introduced, including many cell types such as prokaryotic cells such as *Escherichia coli or Bacillus subtilis,* such as fungal cells such as yeast cells or *Aspergillus,* such as insect cells such as S2 *Drosophila* cells or Sf9, or animal cells such as fibroblasts, CHO cells, COS cells, NSO cells, HeLa cells, BHK cells, HEK 293 cells or human cells.

The term "effective amount" refers to a dose that can achieve a treatment, prevention, alleviation, and/or amelioration of a disease or condition described herein in a subject.

The term "disease and/or condition" refers to a physical state of the subject that is associated with the disease and/or condition described herein.

The term "subject" can refer to a patient or other animal that receives the pharmaceutical composition of the invention to treat, prevent, ameliorate and/or alleviate the disease or condition of the invention, particularly a mammal, such as human, dog, monkey, cow, horse, etc.

In the present invention, the concentration unit µM represents µmol/L, mM represents mmol/L, and nM represents nmol/L, unless otherwise specified.

In the present invention, when the administration dosage in a cell is mentioned, unless otherwise specified, it generally means the final concentration of the drug after administration.

When the term "amino acid" or a specific amino acid name is mentioned in the present invention, it means an L-form amino acid unless otherwise specified.

### Advantageous effects of the invention

The present invention has obtained a novel α-conotoxin (named α-CTx TxID as a new mutant), which is a strong blocker of α3β4 nAChR, and is also the most active α3β4 nAChR blocker discovered so far; in addition, it also has a strong or certain blocking effect on α6/α3β4 nAChR, but has no or extremely weak blocking activity on other nAChR subtypes. There are very few ligand materials that effectively distinguish between two similar subtypes, α3β4 and α6β4* nAChRs. Some of the new TxID mutants of the present invention have a good discrimination between the two subtypes α3β4 and α6β4* nAChRs (more than 45 times, even completely distinguishable), for example, those shown in SEQ ID NO: 7, 19, 24, 29 in Tables 1-3, that is, 7.TxID[S9A], 19.TxID[S9R], 24.TxID[S9K] and 29.TxID[14D]. Thus, the new mutants of α-conotoxin TxID series are a novel tool for the study of the structure and function of α3β4 or α6β4* nAChRs, and contribute to the development of therapeutic drugs against addiction, pain, cancer, depression, fear and the like.

### DRAWINGS

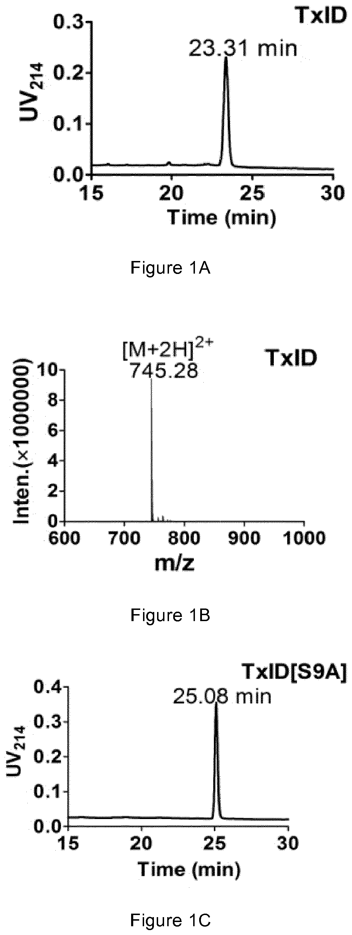

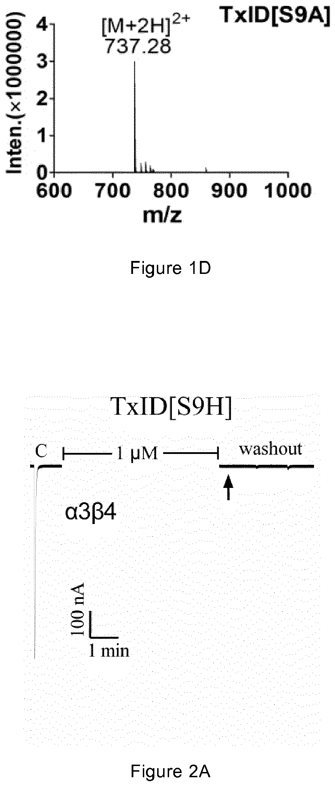

Figure 1: HPLC chromatogram and mass spectrum of TxID and TxID[S9A]. Figure 1A, HPLC chromatogram of TxID with a peak time of 23.31 min. Figure 1B, ESI-MS mass spectrum of TxID, actually measured molecular weight of 1488.56 Da, consistent with the theoretical value. Figure 1C, HPLC chromatogram of TxID[S9A] with a peak time of 25.08 min. Figure 1D, ESI-MS spectrum of TxID[S9A], actually measured molecular weight of 1472.56 Da, consistent with the theoretical value.

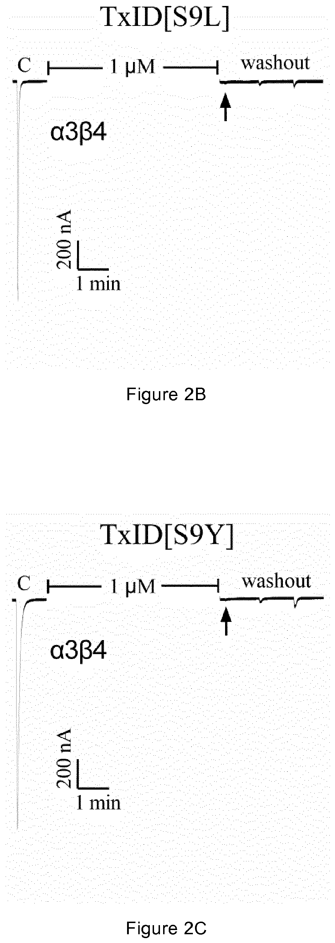

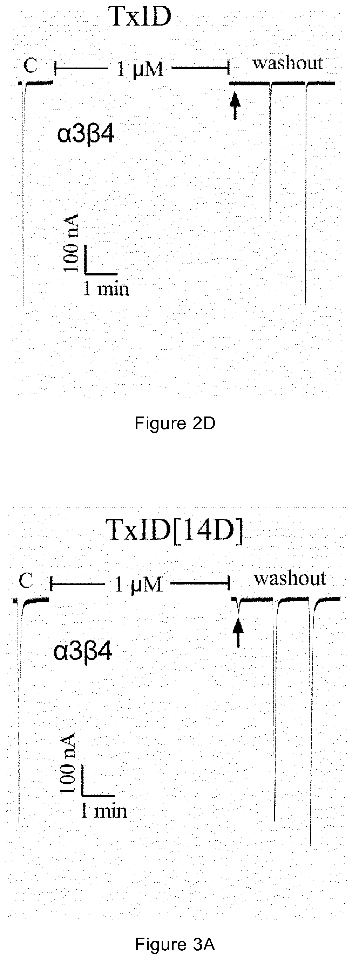

Figure 2: Figures 2A-2D show the strong blocking activities of 1 µM of TxID, TxID[S9H], TxID [S9L] or TxID [S9Y] to rat α3β4 nAChRs current, respectively.
   Rat α3β4 nAChRs were expressed in Xenopus oocytes, and the cell membrane was clamped at -70 mV, giving an Ach pulse of 1 s every minute. In each figure, a representative current trace of one polypeptide on one oocyte is shown. After obtaining the control current, 1 µM of toxin peptide was added, and the first Ach pulse current after 5 min incubation was the current trace of the peptide affecting the receptor, indicated by arrows in the figure. The polypeptide is then eluted, and the magnitude of the current generated by the Ach pulse during the elution and its trace are also measured simultaneously. "C" in the figure indicates the control current generated by ACh excitation. The identification in the following current trace diagram is the same as this description.
Figure 3: Figures 3A-3D show the blocking effects of 1 µM of TxID[14D], TxID[14H], TxID[I14L] or TxID[S9(D-Arg)] on rat α3β4 nAChRs current, respectively.
Figure 4: Figures 4A-4D show the blocking effects of 1 µM of TxID[S9(D-Ser)], TxID[S9A], TxID[S9Abu] or TxID[S9F] on rat α3β4 nAChRs current, respectively.
Figure 5: Figures 5A-5D show the blocking effects of 1 µM of TxID[S9K], TxID[S9T], TxID[S12Y] or TxID[S9R] on rat α3β4 nAChRs current, respectively.
Figure 6: Figures 6A-6E show that 10 µM of TxID[10R], TxID[H5W,S9A], TxID[H5W], TxID[M11H] or TxID[10R] have no blocking effects on the current of rat α3β4 nAChRs.
Figure 7: Figures 7A-7D show the blocking effects of 1 µM of TxID[S9Abu], TxID[S9F], TxID or TxID[I14L] on rat α6/α3β4 nAChRs current, respectively.
Figure 8: Figures 8A-8D show the blocking effects of 1 µM of TxID[S9(D-Arg)], TxID[S9A], TxID[S7H] or TxID[S9L] on rat α6/α3β4 nAChRs currents, respectively..
Figure 9: Figures 9A-9D show the blocking effects of 1 µM of TxID[S9R)], TxID[S9T], TxID[S9Y] or TxID[S12Y] on rat α6/α3β4 nAChRs current, respectively.
Figure 10: Figures 10A-10D show that 10 µM of TxID[9R], TxID[10R], TxID[H5W,S9A], or TxID[H5W] have no blocking effect on the current of rat α6/α3β4 nAChRs.
Figure 11: Figures 11A-11C show that 10 µM of TxID[S9D], TxID[S9E] or TxID[S9K] have no blocking effect on the current of rat α6/α3β4 nAChRs.

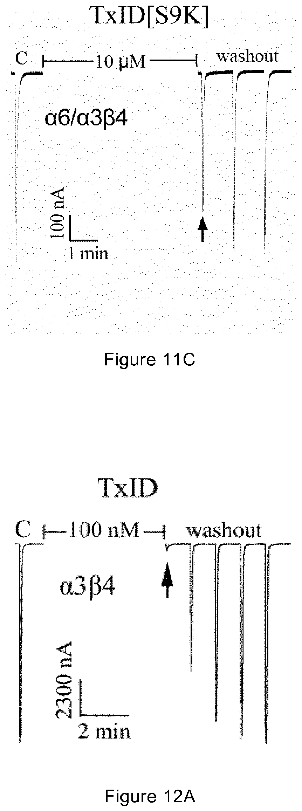

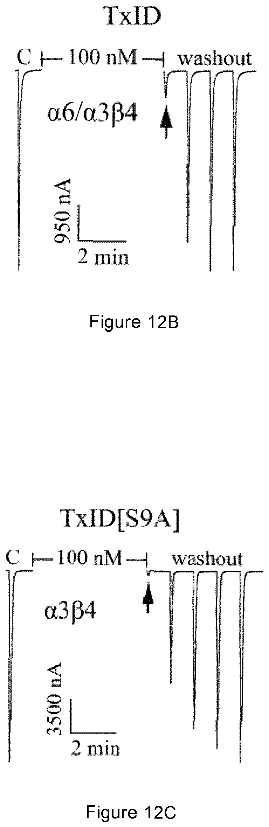

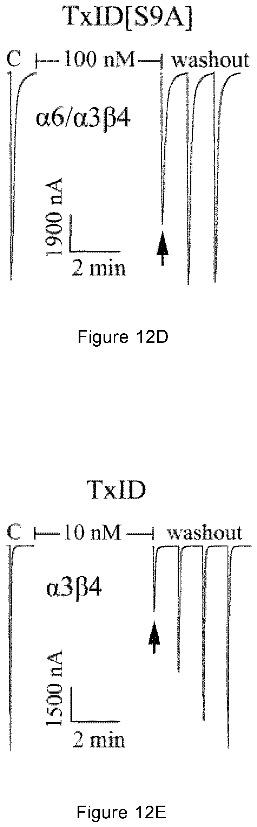

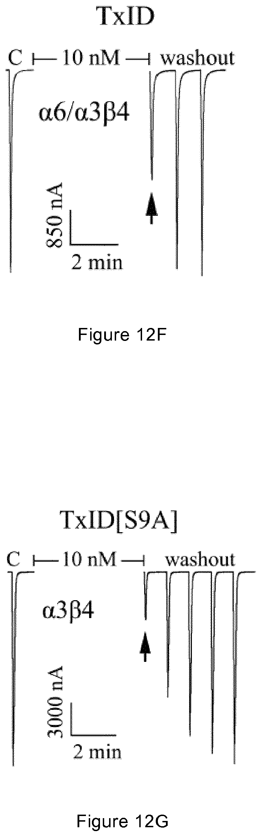

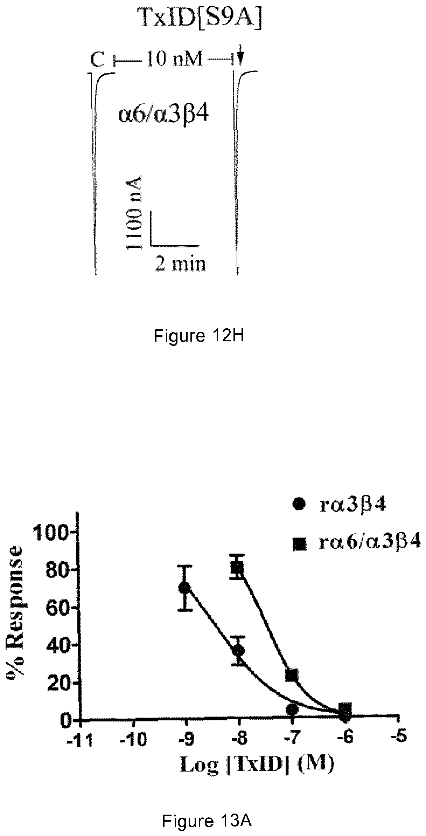

Figure 12: Effects of TxID or TxID[S9A] at 100 nM or 10 nM on the current of rat α3β4 and α6/α3β4 nAChRs both shows the different degrees of discrimination. Rat α3β4 and α6/α3β4 nAChRs were expressed in Xenopus oocytes, and the cell membrane was clamped at -70 mV, giving an Ach pulse of 1 s every minute. A representative current trace of one polypeptide on one oocyte is shown in the figure. After obtaining the control current, 100 nM or 10 nM of toxin peptide was added, and the first Ach pulse current after 5 min incubation was the current trace of the polypeptide affecting the receptor, indicated by the arrows in the figure. The polypeptide is then eluted, and the magnitude of the current generated by the Ach pulse during the elution and its trace were also measured simultaneously. "C" in the figure indicates the control current generated by ACh excitation.
Figure 12A shows the effect of 100 nM TxID on the current of rat α3β4 nAChRs.
Figure 12B shows the effect of 100 nM TxID on the current of rat α6/α3β4 nAChRs.
Figure 12C shows the effect of 100 nM TxID[S9A] on the current of rat α3β4 nAChRs.
Figure 12D shows the effect of 100 nM TxID[S9A] on the current of rat α6/α3β4 nAChRs.
Figure 12E shows the effect of 10 nM TxID on the current of rat α3β4 nAChRs.
Figure 12F shows the effect of 10 nM TxID on the current of rat α6/α3β4 nAChRs.
Figure 12G shows the effect of 10 nM TxID[S9A] on the current of rat α3β4 nAChRs.
Figure 12H shows the effect of 10 nM TxID[S9A] on the current of rat α6/α3β4 nAChRs.

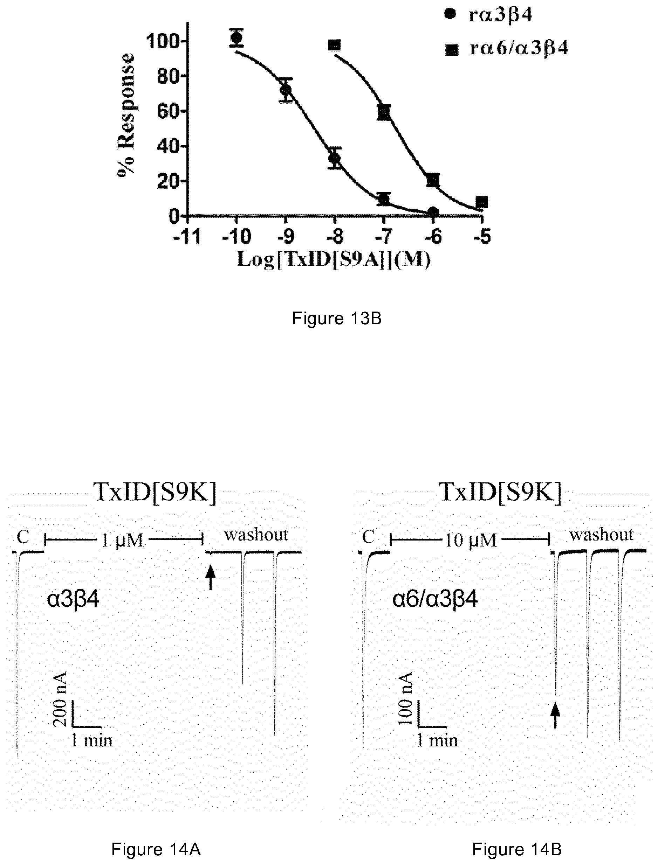

Figure 13 shows the concentration response curves of TxlD or TxID[S9A] to rat α3β4 and α6/α3β4 nAChRs. In the figure, the abscissa is the logarithm of the molar concentration (M) of the polypeptide used; the ordinate is the percentage of dose response (% Response), which is the ratio percentage of the acetylcholine receptor current to the control current at the corresponding concentration of toxin. The corresponding half-blocking dose (IC₅₀) is shown in Table 2. The individual values in the figure are the average values of currents taken from 5-21 Xenopus oocytes. Figure 13A, Concentration dose response curves of TxID to two subtypes of α3β4 nAChRs or α6/α3β4 nAChRs. Figure 13B, Concentration dose response curves of TxID[S9A] to two subtypes of α3β4 nAChRs and α6/α3β4 nAChRs.
Figure 14: Figure 14A shows the effect of 1 µM TxID[S9K] on the current of rat α3β4 nAChRs. Figure 14B shows the effect of 10 µM TxID[S9K] on the current of rat α6/α3β4 nAChRs. Figures 14A and 14B show that TxID[S9K] has a very high degree of discrimination between the two subtypes of α3β4 and α6/α3β4 nAChRs which are extremely similar.
Figure 15 shows the comparison of secondary chemical shift (ordinate) analysis of TxID[S9A] (isomer 1) and TxID (isomer 1).
   The amino acid sequences of TxID[S9A] and TxID are shown in the figure. The amino acid S in parentheses indicates that the serine (Ser) at position 9 of TxlD is substituted with alanine (Ala, A) and then mutated into mutant TxID[S9A]. The black bar in the figure represents TxID[S9A], and the gray bar represents TxID.

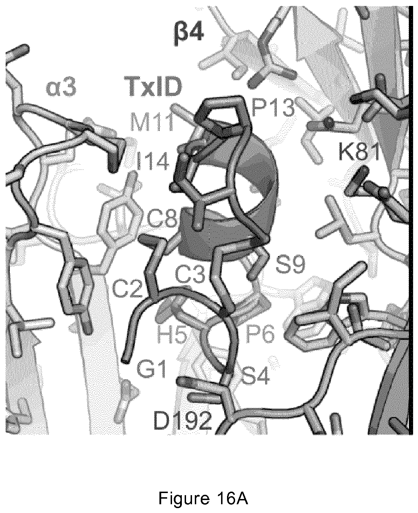

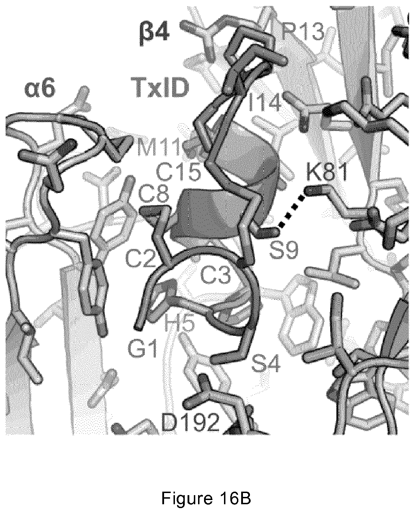

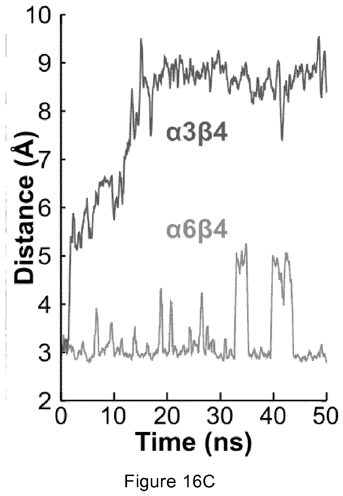

Figure 16: Figure 16A, Molecular binding model of TxlD to α3β4 acetylcholine receptor.
Figure 16B, Molecular binding model of TxID to α6β49 acetylcholine receptor.
In Figures 16A and 16B, the a3 subunit is shown in pink, the a6 subunit is shown in blue, and the β4 subunit is shown in green. Hydrogen bonds are formed between Ser-9 and Lys-81, indicated by dashed lines. The amino acid numbers on the β4 subunit are numbered according to the full length of the rat β4 subunit precursor sequence (UniProt identifier P12392).
Figure 16C shows the results of molecular dynamics (MD) simulations of TxID in 50 ns. The distance between the Ser-9 side chain hydroxyl group of the TxID and the β4 Lys-81 side chain nitrogen atom is shown as a function of time.

### Specific Models for Carrying Out the Invention

Embodiments of the present invention will be described in detail below with reference to the examples. Those skilled in the art will appreciate that the following examples are merely illustrative of the invention and are not to be considered as limiting the scope of the invention. In the examples, when specific techniques or conditions are not indicated, they are carried out in accordance with the techniques or conditions described in the literature in the field (for example, refer to J. Sambrook et al., Huang Peitang et al., Molecular Cloning Experimental Guide, Third Edition, Science Press) or in accordance with the product manual. When the reagents or instruments used are not indicated by the manufacturer, they are conventional products that can be obtained commercially.

### Example 1: Sequence design and artificial synthesis of a novel mutant of α-conotoxin TxID

Based on the α-conotoxin TxID mature peptide (the amino acid sequence thereof is shown in SEQ ID NO: 1 in Table 1 below, and its C-terminal was amidated), the inventors creatively designed a series of new polypeptide mutants. The amino acid sequences thereof are shown in SEQ ID NO: 2-37 in Table 1 below.

**Table 1. Sequences of α-conotoxin TxID and its mutants**

| SEQ ID NO: | Name | Sequence ^{a} | SEQ ID NO: | Name | Sequence ^{a} |
|---|---|---|---|---|---|
| 1 | TxID | GCCSHPVCSAMSPIC* | 20 | TxID-[S9Y] | GCCSHPVCYAMSPIC* |
| 2 | TxID[G1A] | ACCSHPVCSAMSPIC* | 21 | TxID-[S9T] | GCCSHPVCTAMSPIC* |
| 3 | TxID[S4A] | GCCAHPVCSAMSPIC* | 22 | TxID-[S12Y] | GCCSHPVCSAMYPIC* |
| 4 | TxID[H5A] | GCCSAPVCSAMSPIC* | 23 | TxID-[I14L] | GCCSHPVCSAMSPLC* |
| 5 | TxID[P6A] | GCCSHAVCSAMSPIC* | 24 | TxID-[S9K] | GCCSHPVCKAMSPIC* |
| 6 | TxID[V7A] | GCCSHPACSAMSPIC* | 25 | TxID-[S9L] | GCCSHPVCLAMSPIC* |
| 7 | TxID[S9A] | GCCSHPVCAAMSPIC* | 26 | TxID-[S9F] | GCCSHPVCFAMSPIC* |
| 8 | TxID[M11A] | GCCSHPVCSAASPIC* | 27 | TxID-[14H] | GCCSHPVCSAMSPHIC* |
| 9 | TxID[S12A] | GCCSHPVCSAMAPIC* | 28 | TxID-[S9(D-Arg)] | GCCSHPVC(D-Arg)AMSPIC* |
| 10 | TxID[P13A] | GCCSHPVCSAMSAIC* | 29 | TxID-[14D] | GCCSHPVCSAMSPDIC* |
| 11 | TxID[I14A] | GCCSHPVCSAMSPAC* | 30 | TxID-[S9(D-Ser)] | GCCSHPVC(D-Ser)AMSPIC* |
| 12 | TxID[M11I] | GCCSHPVCSAISPIC* | 31 | TxID-[S9E] | GCCSHPVCEAMSPIC* |
| 13 | TxID[S9A, M11L] | GCCSHPVCAALSPIC* | 32 | TxID-[S9D] | GCCSHPVCDAMSPIC* |
| 14 | TxID[I14R] | GCCSHPVCSAMSPRC* | 33 | TxID-[M11H] | GCCSHPVCSAHSPIC* |
| 15 | TxID[I14Y] | GCCSHPVCSAMSPYC* | 34 | TxID-[H5W, S9A] | GCCSWPVCAAMSPIC* |
| 16 | TxID[I14D] | GCCSHPVCSAMSPDC* | 35 | TxID-[H5W] | GCCSWPVCSAMSPIC* |
| 17 | TxID-[S9Abu] | GCCSHPVCBAMSPIC* | 36 | TxID-[9R] | GCCSHPVCRSAMSPIC* |
| 18 | TxID-[S9H] | GCCSHPVCHAMSPIC* | 37 | TxID-[10R] | GCCSHPVCSRAMSPIC* |
| 19 | TxID-[S9R] | GCCSHPVCRAMSPIC* | | | |

| | | | | | |
|---|---|---|---|---|---|
| ^{a} Mutation sites for each mutant are underlined. B in the sequence represents 2-aminobutyric acid (Abu). * indicates C-terminal amidation. | | | | | |

Linear peptides of the polypeptides listed in Table 1 were artificially synthesized by the Fmoc method. The specific method is as follows:
The resin peptides were artificially synthesized by Fmoc chemical method, and the resin peptides could be synthesized by a peptide synthesizer or a manual synthesis method. In addition to cysteine, the remaining amino acids were protected with standard side chain protecting groups. The -SH groups of the first and third cysteines (Cys) of each polypeptide were protected by Trt (S-trityl), and the -SH groups of the second and fourth cysteines were protected by Acm (S-acetamidomethyl), and the disulfide linkages after oxidative folding were Cys1-Cys3 and Cys2-Cys4, i.e., Cys (1-3, 2-4). The synthesis procedure was as follows: a linear peptide was synthesized on the ABI Prism 433a polypeptide synthesizer by Fmoc and FastMoc methods in solid phase synthesis. The side chain protecting groups of Fmoc amino acids were: Pmc (Arg), Trt (Cys), But (Thr, Ser, Tyr), OBut (Asp), Boc (Lys). Using Fmoc HOBT DCC method, the resin and Fmoc amino acids were amidated by Rink, and the synthesis steps were carried out with reference to the instrument synthesis manual. In order to complete the reaction, the piperidine deprotection time and the coupling time were appropriately extended, and the refractory amino acids were double-coupled to obtain a resin peptide. The linear peptide was cleaved from the resin with reagent K (trifluoroacetic acid /water / ethanedithiol / phenol / thioanisole; 90:5:2.5:7.5:5, v/v/v/v/v) and precipitated and washed with ice diethyl ether to recycle a crude linear peptide.

Preparative reverse HPLC C18 column (Vydac) was used for purification, in which the elution gradient was 10-40% B90 in 0-20 min. The solvent B90 was composed of 90% ACN (acetonitrile), 0.5% TFA (trifluoroacetic acid), and balance of pure water; the solvent A was an aqueous solution of 0.65% TFA. UV absorption analysis was performed at a wavelength of 214 nm. The purified linear peptide was subjected to purity detection using an analytical HPLC C18 column (Vydac), and the elution gradient was the same as above. It had a purity of over 95% and was used for oxidative folding.

According to references (Dowell, C.; Olivera, B.M.; Garrett, J.E.; Staheli, S.T.; Watkins, M.; Kuryatov, A.; Yoshikami, D.; Lindstrom, J.M.; McIntosh, J.M., Alpha-conotoxin PIA is selective for alpha6 subunit-containing nicotinic acetylcholine receptors. The Journal of neuroscience. 2003, 23 (24), 8445-52), a two-step oxidative folding reaction of the above linear peptide was described as follows:
The first pair of disulfide bonds were first formed between the two cysteines of the Trt protecting groups by potassium ferricyanide oxidation (20 mM potassium ferricyanide, 0.1 M Tris, pH 7.5, 30 min). The monocyclic peptide was purified by reverse-phase HPLC C18 column (Vydac) and then oxidized with iodine (10 mM iodine in H₂O: trifluoroacetic acid: acetonitrile (78:2:20 by volume, 10 min), the Acm on other two cysteines were removed, and the second pair of disulfide bonds were formed between these two cysteines. The bicyclic peptide was purified by reverse phase HPLC C18 column (Vydac), and the linear gradient of elution was still 10-40% B90 within 0-20 min. Ultraviolet absorption analysis was carried out at a wavelength of 214 nm. Thus, an α-conotoxin with disulfide bonds formed in oriented manner between the corresponding cysteines in order from the N-terminus to the C-terminus was obtained.

The purified peptides with 2 pairs of disulfide bonds were tested for purity and molecular weight by HPLC and mass spectrometry, and the results were all correct. The HPLC chromatographic conditions were as follows: Vydac C18 HPLC reverse phase analytical column, gradient elution in 20 minutes with B solution from 10% to 40% and solution A from 90% to 60%, in which the solution A was 0.65% trifluoroacetic acid (TFA), the B solution was an aqueous solution of 0.5% TFA and 90% acetonitrile. The UV analysis wavelength was 214 nm, and the peak time of TxIC, i.e., the retention time, was 23.366 minutes.

For example, Figures 1A-1D show HPLC chromatograms and mass spectra of TxID and TxID[S9A]. The peak time of TxID was 23.31 min (Figure 1A) and was identified as correct by mass spectrometry (ESI-MS) (Figure 1B). The monoisotopic mass of TxID after oxidative folding was consistent with the measured molecular weight: the theoretical molecular weight of TxID was 1488.559 Da, and the molecular weight of TxID was 1488.56 Da. The peak time of TxID[S9A] was 25.08 min (Figure 1C) and was also confirmed by ESI-MS mass spectrometry (Figure 1D). The monoisotopic mass of the oxidatively folded TxID[S9A] was also consistent with the measured molecular weight: the theoretical molecular weight of TxID[S9A] was 1472.564 Da, and the measured molecular weight of TxID[S9A] was 1472.56 Da.

Through the above steps, all the 37 polypeptides listed in Table 1 were correctly synthesized, and oxidatively folded peptides with disulfide linkages of Cys (1-3, 2-4) were formed, which could be used for subsequent activity research and structural analysis.

The concentrations of the polypeptide were determined by colorimetry at a wavelength of 280 nm, and the concentrations and masses of the polypeptides were calculated according to the Beer-Lambert equation, and used in the experiments in the following examples.

### Example 2: Experiment of α-conotoxin TxID new mutant specifically blocking α3β4 nAChR subtype

Referring to the methods in the literature (Luo S, Zhangsun D, Zhu X, Wu Y, Hu Y, Christensen S, Harvey PJ, Akcan M, Craik DJ, McIntosh JM. Characterization of a novel α-conotoxin TxID from Conus textile that potently blocks rate α3β4 nicotinic acetylcholine receptors. Journal of Medicinal Chemistry. 2013, 56: 9655-9663. Azam L, Yoshikami D, McIntosh JM. Amino acid residues that confer high selectivity of the alpha6 nicotinic acetylcholine receptor subunit to alpha-conotoxin MII[S4A,E11A,L15A]. J Biol Chem. 2008;283(17):11625-32.), as well as the specification of in vitro transcription kit (mMessage mMachine in vitro transcription kit (Ambion, Austin, TX)), various rat neural nAChRs subtypes (α3β4, α6/α3β4, α9α10, α4β2, α4β4, α3β4, α2β2, α2β4, α7), human α3β4 and α6/α3β4, and mouse muscular nAChRs (α1β1δε) cRNA were prepared, and their concentrations were measured and calculated by OD values at UV 260 nm. Xenopus laveis were dissected and oocytes (frog eggs) were collected, cRNA was injected into frog eggs with 5-10 ng cRNA per subunit. Frog eggs were cultured in ND-96. cRNA was injected within 1-2 days after the collection of frog eggs, and voltage clamp for nAChRs was recorded within 1-4 days after injection.

One frog egg injected with cRNA was placed in a 30 µL Sylgard recording groove (depth 4mm × diameter 2mm), gravity infusion was performed with a ND96 perfusate (96.0 mM NaCl, 2.0 mM KCI, 1.8 mM CaCl₂, 1.0 mM MgCl₂,5 mM HEPES, pH 7.1-7.5) containing 0.1 mg/ml BSA (bovine serum albumin), or a ND96 (ND96A) containing 1 mM atropine, in a flow rate of 1ml/min. All conotoxin solutions also contained 0.1 mg/ml BSA to reduce non-specific adsorption of toxins, a switch valve (SmartValve, Cavro Scientific Instruments, Sunnyvale, CA) was used to switch between infusion of toxins and acetylcholine (ACh), and a series of three-way solenoid valves (model 161TO31, Neptune Research, Northboro, MA) were used to freely switch between perfusion ND96 and ACh. Online recordation was performed when the Ach-gated current was set at "slow" clamp by a two-electrode voltage-clamp amplifier (model OC-725B, Warner Instrument Corp., Hamden, CT) and the clamp gain at the maximum (x2000) position. A glass electrode was drawn from glass capillary (fiber-filled borosilicate capillaries, WPI Inc., Sarasota, FL) with 1 mm outer diameter x 0.75 inner diameter mm, and filled with 3M KCI as a voltage and current electrode. The membrane voltage was clamped at -70 mV. The entire system was controlled and recorded by a computer. The ACh pulse was automatic perfusion with ACh for 1 s every 5 min. The concentrations of ACh were 10 µM for the muscular nAChRs and the neuronal α9α10 nAChRs, 200 µM for the a7 of expression neuronal nAChRs, and 100 µM for other subtypes. The current responses and the current traces of at least 5 eggs expressing a certain subtype to different toxin concentrations were recorded.

The current data of the test were statistically analyzed by GraphPad Prism software (San Diego, CA), and a dose-response curve was drawn to calculate various parameters of conotoxin, such as half-blocking concentration IC₅₀ and the like, about toxin blocking nAChRs.

The experimental results are shown in Table 2 below. The ratios of the blocking activities of these mutants to α3β4 nAChR to that of TxID are also summarized in Table 2.

**Table 2: Blocking activities of α-conotoxin TxID and its mutants on rat α3β4 and α6/α3β4 acetylcholine receptor subtypes (half-blocking dose IC₅₀).**

| SEQ ID NO: | Polypeptide name^{a} | α3β4 IC₅₀, nM^{c} | α3β4 IC₅₀ ratio^{d} | α6/α3β4 IC₅₀, nM^{c} | α6/α3β4 IC₅₀ ratio^{e} | IC₅₀ ratio^{f} of α6/α3β4 vs. α3β4 |
|---|---|---|---|---|---|---|
| 1 | TxID | 3.64 (1.8-7.3) | 1.0 | 33.9 (23.6-48.7) | 1.0 | 9.3 |
| 2 | TxID[G1A] | 61.9 (32.4-118.0) | 17.0 | 278 (154-503) | 8.2 | 4.5 |
| 3 | TxID[S4A] | 10.8 (8.6-13.4) | 3.0 | 64.1 (41.6-98.7) | 1.9 | 5.9 |
| 4 | TxID[H5A] | >10000^{b} | - | >10000^{b} | - | - |
| 5 | TxID[P6A] | >10000^{b} | - | >10000^{b} | - | - |
| 6 | TxID[V7A] | >10000^{b} | - | >10000^{b} | - | - |
| 7 | TxID[S9A] | 3.89 (2.5-5.9) | 1.1 | 178.1 (137.0-231.5) | 5.2 | 45.8 |
| 8 | TxID[M11A] | >10000^{b} | - | >10000^{b} | - | - |
| 9 | TxID[S12A] | 17.4 (8.6-3.5) | 4.8 | 39.3 (25.6-59.8) | 1.2 | 2.3 |
| 10 | TxID[P13A] | >10000^{b} | - | >10000^{b} | - | - |
| 11 | TxID[I14A] | 16.1 (9.1-28.5) | 4.4 | 45.8 (33.7-62.2) | 1.3 | 2.8 |
| 12 | TxID[M11I] | 74.9 (55.0-102.1) | 20.6 | 50.4 (31.3-81.2) | 1.5 | 0.7 |
| 13 | TxID[S9A, M11I] | 30.7 (14.1-66.8) | 8.4 | 101.0 (55.8-183.2) | 3.0 | 0.3 |
| 14 | TxID[I14R] | 9.2 (5.0-16.9) | 2.5 | 67.4 (42.8-106) | 2.0 | 7.3 |
| 15 | TxID[I14Y] | 10.0 (6.4-15.6) | 2.7 | 38.7 (23.0-65.0) | 1.1 | 3.9 |
| 16 | TxID[I14D] | 9.8 (6.6-14.4) | 2.7 | 40.8 (32.9-50.5) | 1.2 | 4.2 |
| 17 | TxID[S9Abu] | 1.87(1.48-2.37) | 0.5 | 4.91 (3.65-6.6) | 0.1 | 2.6 |
| 18 | TxID[S9H] | 2.61(2.27-2.99) | 0.7 | 14.69(10.57-20.41) | 0.4 | 5.6 |
| 19 | TxID[S9R] | 5.26(3.87-7.13) | 1.5 | 264.1(177.1-393.88) | 7.8 | 50.2 |
| 20 | TxID[S9Y] | 7.66(6.06-9.67) | 2.1 | 17.76(12.42-25.37) | 0.5 | 2.3 |
| 21 | TxID[S9T] | 7.8(6.16-9.87) | 2.2 | 39.06(31.95-47.75) | 1.2 | 5 |
| 22 | TxID[S12Y] | 9.09(7.04-11.73) | 2.5 | 34.23(28.18-41.57) | 1.0 | 3.8 |
| 23 | TxID[I14L] | 9.98(8.33-11.97) | 2.8 | 28.98(22.11-38) | 0.9 | 2.9 |
| 24 | TxID[S9K] | 10.13(8.07-12.72) | 2.8 | >10000^{b} | - | - |
| 25 | TxID[S9L] | 13.11(10.25-16.78) | 3.6 | 16.18(11.34-23.08) | 0.5 | 1.2 |
| 26 | TxID[S9F] | 13.85(10.48-18.3) | 3.8 | 7.11(6.02-8.39) | 0.2 | 0.5 |
| 27 | TxID[14H] | 32.97(24.61-44.18) | 9.2 | 312.02(198.26-491.01) | 9.2 | 9.5 |
| 28 | TxID[S9(D-Arg)] | 47.93(34.65-66.31) | 13.3 | 338.93(250.26-458.88) | 10.0 | 7.1 |
| 29 | TxID[14D] | 50.58(37.49-68.25) | 14.1 | 2258.5(1342.4-3798.94) | 66.6 | 44.7 |
| 30 | TxID[S9(D-Ser)] | 115.88(84.85-158.14) | 32.2 | 395.84(284.2-551.4) | 11.7 | 3.4 |
| 31 | TxID[S9E] | 307.61 (231.28-409.17) | 85.4 | >10000^{b} | - | - |
| 32 | TxID[S9D] | 379.5(230.7-568.3) | 105.4 | >10000^{b} | 303 | - |
| 33 | TxID[M11H] | >10000^{b} | - | 3492.82(1601.66-7611.49) | 103 | - |
| 34 | TxID[H5W, S9A] | >10000^{b} | - | >10000^{b} | - | - |
| 35 | TxID[H5W] | >10000 ^{b} | - | >10000 ^{b} | - | - |
| 36 | TxID[9R] | >10000 ^{b} | - | >10000 ^{b} | - | - |
| 37 | TxID[10R] | >10000 ^{b} | - | >10000 ^{b} | - | - |

| | | | | | | |
|---|---|---|---|---|---|---|
| ^{a} Cysteine mode is C₁C₂-C₃-C₄, and disulfide bond mode is 1-3, 2-4. ^{b} Blocking current is less than 50% at a high concentration of 10 µM. ^{c} Number in parentheses indicates the range of half-blocking dose (IC₅₀) for 95% confidence interval. ^{d} Ratio of the IC₅₀ of TxID mutant to α3β4 nAChR subtype to the IC₅₀ of wild-type TxID to α3β4 nAChR subtype. ^{e} Ratio of the IC₅₀ of TxID mutant to α6/α3β4 nAChR subtype to the IC₅₀ of wild-type TxID to α6/α3β4 nAChR subtype. ^{f} Ratio of the IC₅₀ of each peptide to α6/α3β4 nAChR subtype to its IC₅₀ to α3β4 nAChR subtype. | | | | | | |

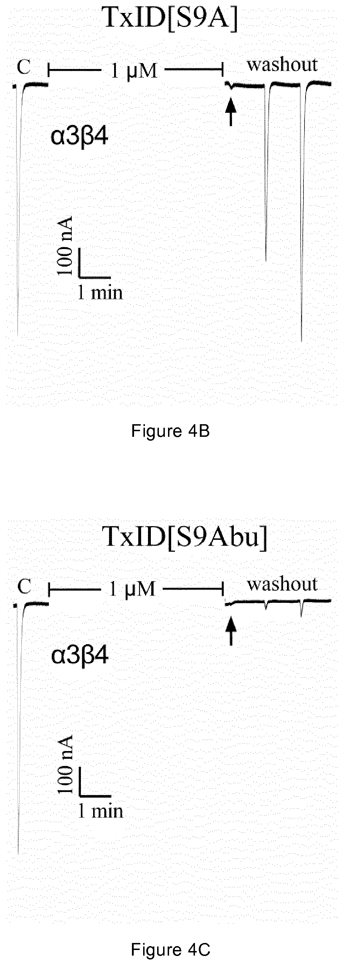

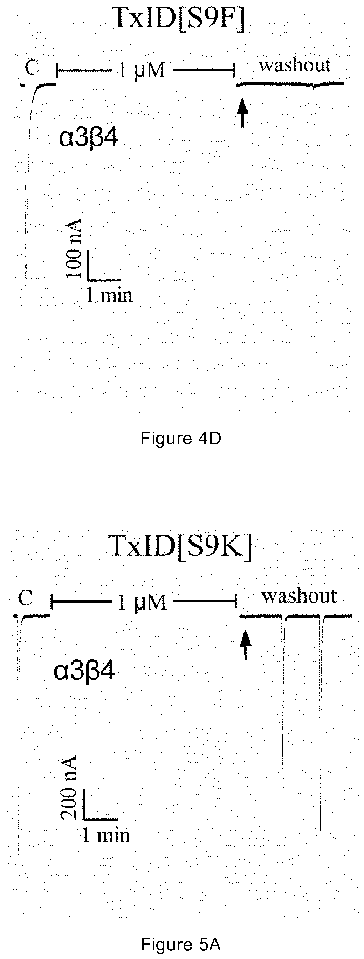

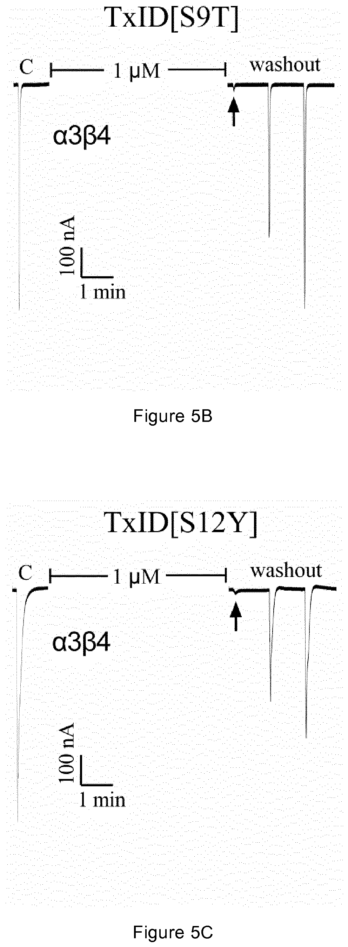

The results show:
The 26 mutants shown in SEQ ID NO: 2-3, 7, 9, 11-32 (prepared in Example 1) all had nanomolar blocking activity to rat α3β4 nAChR (see Table 2, and Figures 2A-2D, Figures 3A-3D, Figures 4A-4D, and Figures 5A-5D), and their half-blocking doses (IC₅₀) varied from 1.87 nM to 379.5 nM (Table 2). Among them, most of the mutants (23 polypeptides in total) maintained strong blocking activity against α3β4 nAChR, and their IC₅₀ values were all below 100 nM; and 19 polypeptides had stronger blocking activity against α3β4 nAChR, and their IC₅₀ were all below 36 nM.

Most of the mutants had a strong blocking activity against α3β4 nAChR, their IC₅₀ values were close to that of TxID and all below 10 nM, and some of them were even stronger than the blocking activity of TxID (IC₅₀, 3.6 nM); for example, the IC₅₀ values of SEQ ID NO: 17 TxID[S9Abu] and SEQ ID NO: 18 TxID[S9H] were only 1.87 nM and 2.61 nM, respectively (Table 2), and they were the specific blockers to α3β4 nAChR with the strongest blocking activity discovered so far.

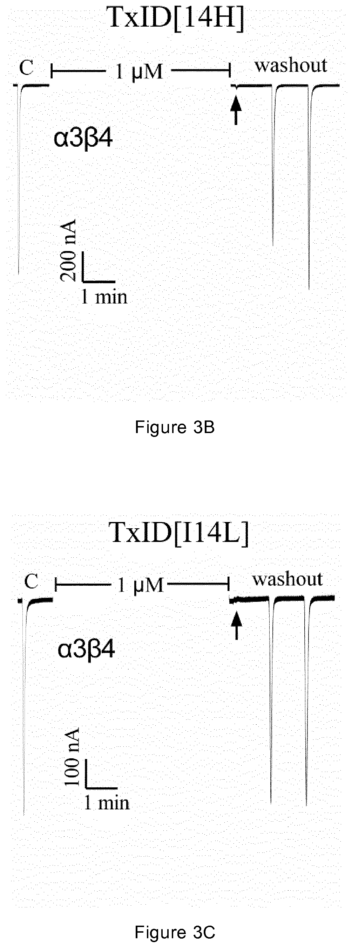

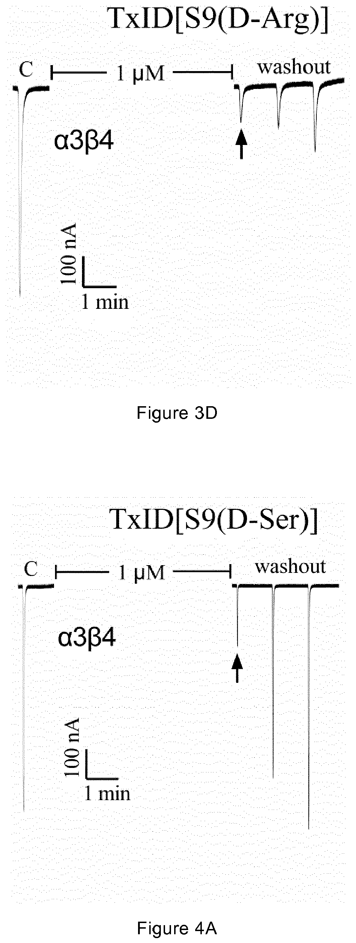

The elution rates of TxID and its new mutants after blocking the α3β4 nAChR current were different (Figures 2A-2D, Figures 3A-3D, Figures 4A-4D, Figures 5A-5D). As shown in Figures 2A-2D, after 1 µM of the new mutants TxID[S9H], TxID[S9L] and TxID[S9Y] blocked the α3β4 nAChR current, the elution was very slow, and the current after 2 min of elution was almost 0 nA. However, after blocking with 1 µM wild-type TxID, its current returned to the level of control current "C" within 2 min of elution. Also, after TxID[S9Abu] and TxID[S9F](4A-4D) blocked the α3β4 nAChR current, the elution was also very slow. The elution rates of the remaining polypeptides were relatively fast, but the respective elution rates were different (Figures 3A-3D, Figures 4A-4D, Figures 5A-5D). The elution rate reflected an important feature of the binding between polypeptide and receptor, namely the rate at which the reaction binding dissociated.

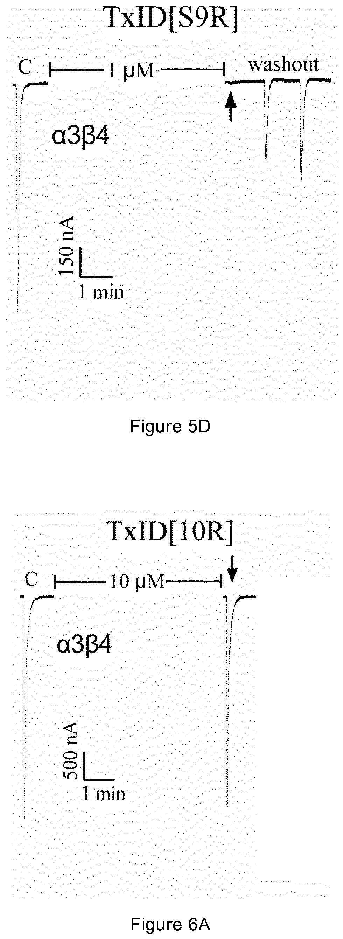

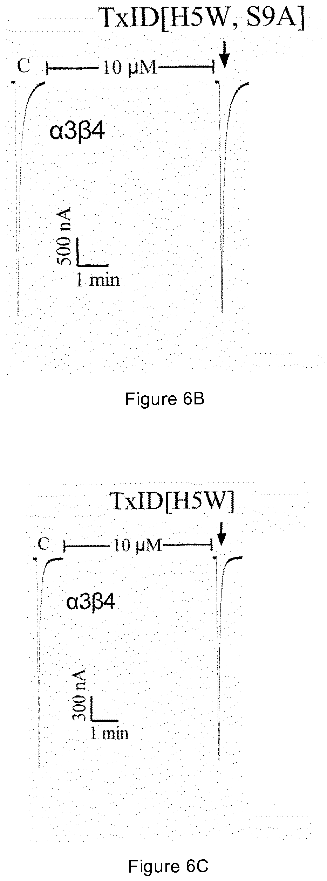

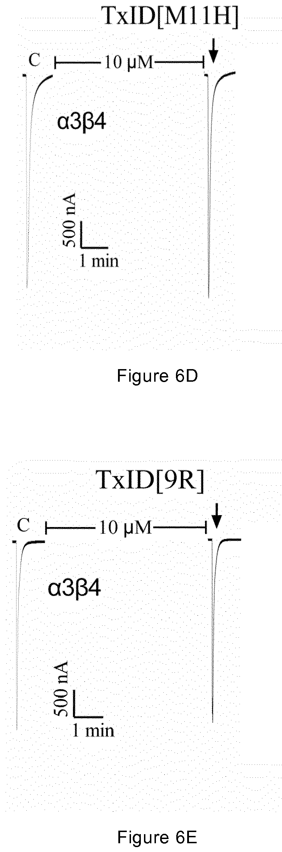

Only the three mutant polypeptides, SEQ ID NO: 30 TxID[S9(D-Ser)], SEQ ID NO: 31 TxID[S9E], SEQ ID NO: 32 TxID[S9D], had an IC₅₀ between 115 and 380 nM. There were also 10 polypeptides, SEQ ID NOs: 4, 5, 6, 8, 10, and 33-37, which lost blocking activity on α3β4 nAChR (Table 2, Figures 6A-6E), and at a high concentration of 10 µM, their blocking activities to α3β4 nAChR current were less than 50% and their IC₅₀ were greater than 10000 nM (see Table 2, and Figures 6A-6E).

In addition, the present inventors also determined the blocking activities of TxlD and its new mutants (such as Table 1) on human α3β4 nAChR, and their activities were similar to those to rat α3β4 nAChR, and there was no significant difference between the two species.

### Example 3: Experiment of α-conotoxin TxID new mutants specifically blocking α6/α3β4 nAChR subtypes

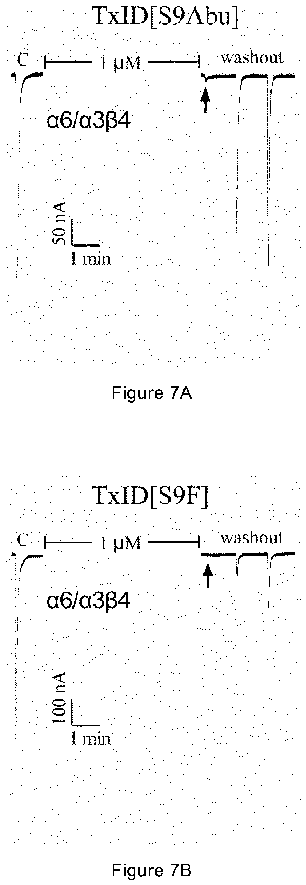

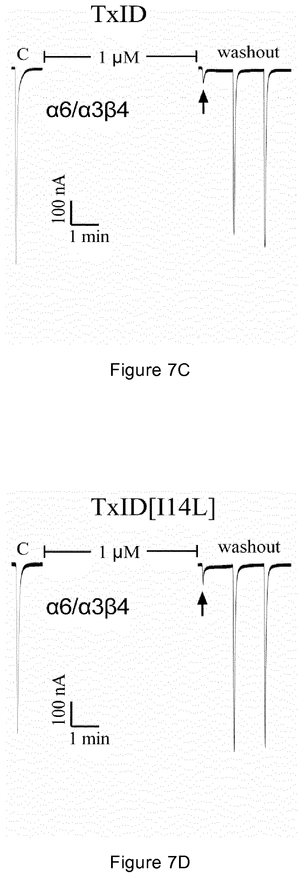

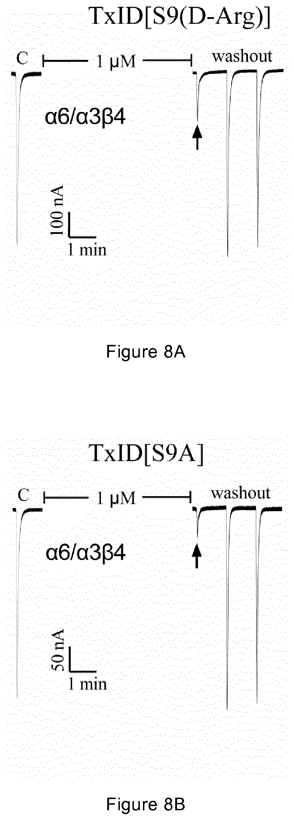

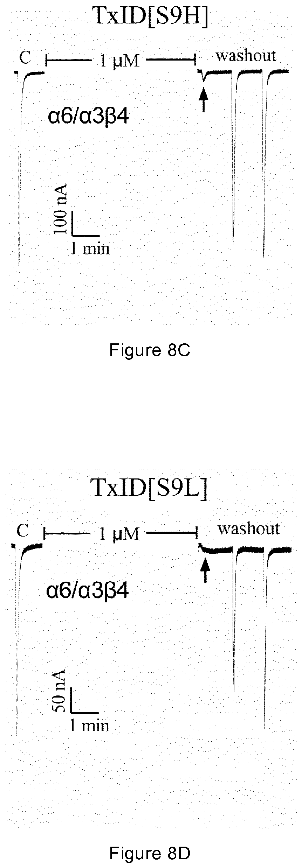

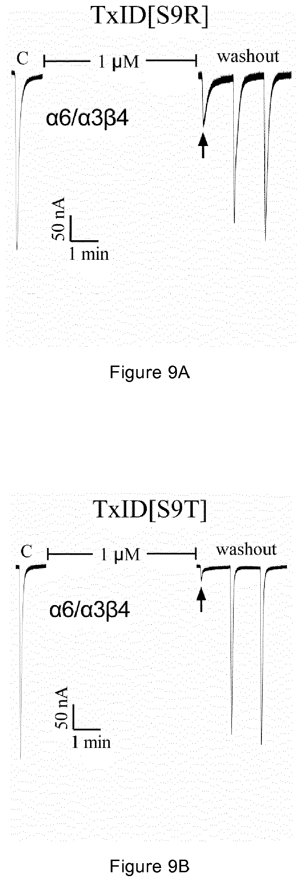

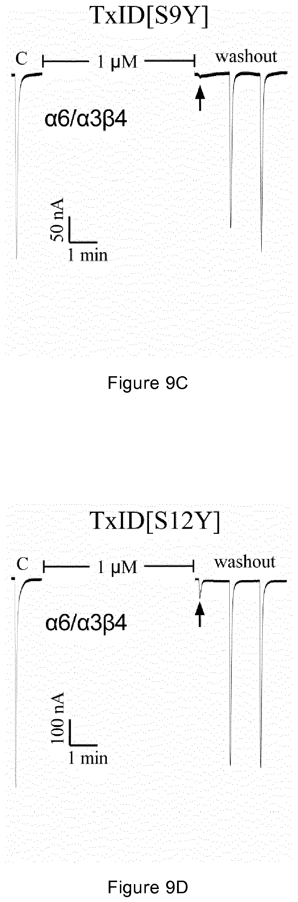

Referring to the electrophysiological method in Example 2, the blocking activities of TxlD and all polypeptides of its new mutant in Table 1 against α6/α3β4 (equivalent to α6β4*, * representing the remaining possible subunits) nAChR were determined, and the results were shown in Table 2, as well as Figures 7A-7D, Figures 8A-8D, Figures 9A-9D, Figures 10A-10D, and Figures 11A-11C. The ratios of the blocking activities of these mutants to α6/α3β4 nAChR compared to TxID were also summarized in Table 2.

The results show:
Total of 24 new peptides, SEQ ID NOs: 2-3, 7, 9, 11-23, 25-30 and 33 (prepared in Example 1), had different degrees of blocking effect on rat α6/α3β4 nAChR (please See Table 2, and Figures 7A-7D, Figures 8A-8D and Figures 9A-9D), and half-blocking doses (IC₅₀) ranging from 4.91 nM to 3492.82 nM (Table 2).

Among them, most of the mutants in total of 22 polypeptides maintained blocking activities on α6/α3β4 nAChR, and their IC₅₀ were all below 1000 nM, in which there were 7 polypeptides, namely SEQ ID NO: 2, 7, 13, 19, 27, 28 and 30, presenting IC₅₀ to α6/α3β4 nAChR in a range of 100-1000 nM. There were 15 mutants that had stronger blocking activity on α6/α3β4 nAChR, and their IC₅₀ values were close to that of TxID and all below than 100 nM, and some of them were stronger than the blocking activity of TxID on α6/α3β4 nAChR (IC₅₀, 33.9 nM); for example, the IC₅₀ values of SEQ ID NOS: 20, 23, 25 and 26 were 17.76 nM, 28.98 nM, 16.18 nM and 7.11 nM, respectively (Table 2). There were two mutant polypeptides, SEQ ID NO: 29 and SEQ ID NO: 33, which showed IC₅₀ in a range between 1 µM and 10 µM.

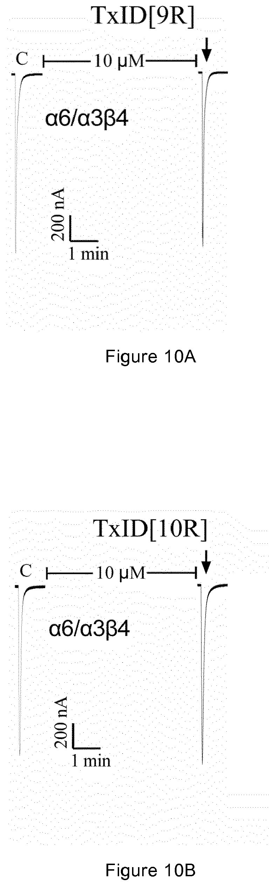

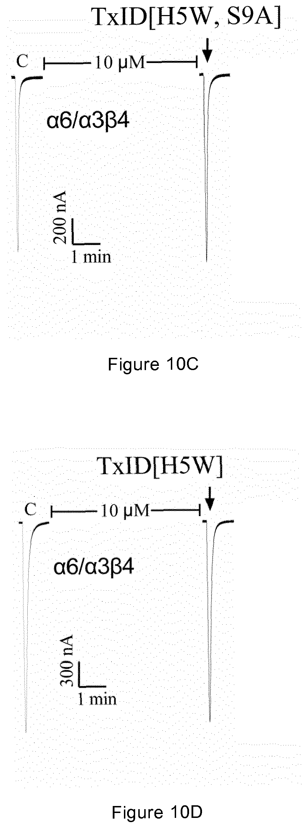

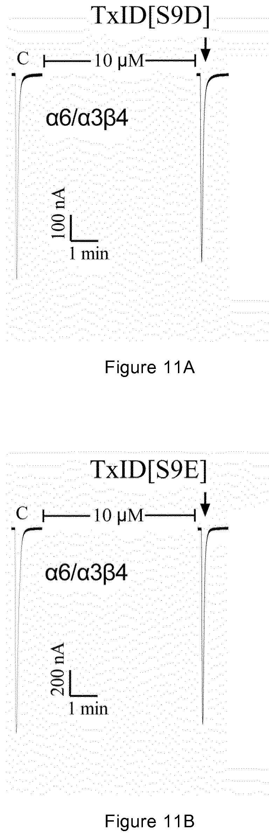

In addition, there were 12 polypeptides, SEQ ID NOs: 4-6, 8, 10, 24, 31-32, 34-37, which almost lost blocking activity against α6/α3β4 nAChR (Table 2, Figures 10A-10D, and Figures 11A -11C), and their IC50 values were greater than 10000 nM (i.e., 10 µM).

The elution rates of TxID and its new mutants after blocking α6/α3β4 nAChR current were different (Figures 7A-7D, Figures 8A-8D, Figures 9A-9D). As shown in Figures 7A-7D, the elution rate of TxID[S9F] was significantly slower than those of other polypeptides. In general, they are similar to the elution rate and peak shape of wild-type TxID after blocking α6/α3β4 nAChR current (Figures 7A-7D, Figures 8A-8D, Figures 9A-9D), and could generally return to the level of the control current "C" after elution for 2 min.

In addition, the inventors also determined the blocking activities of TxlD and its new mutants (as shown in Table 1) on human α6/α3β4 nAChR, indicating that their activities were similar to those on rat α6/α3β4 nAChR, and there were no significant difference between the two species.

### Example 4: Comparison of activities of α-conotoxin TxID new mutants on two subtypes of α3β4 and α6/α3β4 nAChR

The present inventors compared the activities of the α-conotoxin TxID new mutants on the two subtypes of α3β4 and α6/α3β4 nAChR according to the experimental data in Table 2 above, in which the comparison results were expressed by the degrees of discrimination, the degree of discrimination = (IC₅₀ of mutant on rat α6/α3β4)/IC₅₀ of mutant on rat α3β4), and the unit was nM. The results are shown in Table 3 below. In addition, the inventors also list the currently known conotoxins acting on α3β4 nAChRs and their discriminations between two very similar subtypes α3β4 and α6/α3β4 nAChR in Table 3 by literature investigation.

**Table 3. Alpha-conotoxins acting on α3β4 and α6/α3β4 acetylcholine receptor subtypes and their discrimination for the two subtypes.**

| SEQ ID NO: | Name | Sequence ^{a} | Source | α3β4 IC₅₀[nM] | α6/α3β4 IC₅₀[nM] | Discrim ination ^{b} | Selectivity to nAChR subtype | Reference ^{d} |
|---|---|---|---|---|---|---|---|---|
| 1 | TxID | GCCSHPVCSAMSPIC* | *C*. *textile* | 3.64 | 33.9 | 9.3 | α3β4>α6/α3β4 | *Luo* S. *et al*. *2013b* |
| 7 | TxID[S9A] | GCCSHPVCAAMSPIC* | synthetic | 3.89 | 178.1 | 45.8 | α3β4>α6/α3β4 | - |
| 19 | TxID[S9R] | GCCSHPVCRAMSPIC* | Artificially synthesized | 5.26 | 264.1 | 50.2 | α3β4>α6/α3β4 | - |
| 24 | TxID[S9K] | GCCSHPVCKAMSPIC* | Artificially synthesized | 10.13 | >10000^{c} | - | α3β4>α6/α3β4 | - |
| 29 | TxID[14D] | GCCSHPVCSAMSPDIC* | Artificially synthesized | 50.58 | 2258.5 | 44.7 | α3β4>α6/α3β4 | - |
| 17 | TxID[S9Abu] | GCCSHPVCBAMSPIC* | Artificially synthesized | 1.87 | 4.91 | 2.6 | α3β4>=α6/α3β4 | - |
| 18 | TxID[S9H] | GCCSHPVCHAMSPIC* | Artificially synthesized | 2.61 | 14.69 | 5.6 | α3β4>α6/α3β4 | - |
| 31 | TxID[S9E] | GCCSHPVCEAMSPIC* | Artificially synthesized | 307.61 | >10000^{c} | - | α3β4 | - |
| 38 | TP-2212-59 | GCCSHPBCFBZYC* | Artificially synthesized | 2.3 | N.D. | - | α3β4 | *Chang et al*. *2014* |
| 39 | AuIB | GCCSYPPCFATNPDC* | *C*. *aulicus* | 750 | N.D. | - | α3β4 | *Luo* S. *et al*. *1998* |
| 40 | RegIIA | GCCSHPACNVNNPHIC* | *C*. *regius* | 47.3 | 147 | 3.1 | α3β2>α3β4=α6β2>α 7>α6β4 | *Franco et al*. *2012* |
| 41 | RegIIA[N11,12 A] | GCCSHPACNVAAPHIC* | Artificially synthesized | 370 | 5100 | 13.8 | α3β4>α6/α3β4* | *Kompella et al*. *2015* |
| 42 | PelA | GCCSHPACSVNHPELC* | *C. pergrandis* | 480 | 1500 | 3.1 | α9α10>α3β2>α6β2> α3β4>α7 | *McIntosh et al*. *2005* |
| 43 | PIA | RDPCCSNPVCTVHNPQIC* | *C. purpurascens* | 518 | 30 | 17.3 | α6β2*>>α6β4≈α3β2 >α3β4 | *Dowell et al*. *2003* |
| 44 | BuIA | GCCSTPPCAVLYC* | *C*. *bullatus* | 27.7 | 2.1 | 13.2 | α6α3*>α3β2>α3β4> α4β4>α2* | *Azam et al*. *2005)* |

| | | | | | | | | |
|---|---|---|---|---|---|---|---|---|
| ^{a} B represents 2-aminobutyric acid (Abu). Z represents pentane. ^{b} Ratio between the IC₅₀ of each polypeptide to α6/α3β4 nAChR subtype and the IC₅₀ to α3β4 nAChR subtype. ^{c} Blocking less than 50% of current at a high concentration of 10 µM. * indicates C-terminal amidation. N.D. indicates not determined, no reference value. ^{d} References: *Luo S. et al. 2013b:* Luo S, Zhangsun D, Zhu X, Wu Y, Hu Y, Christensen S, Harvey PJ, Akcan M, Craik DJ, McIntosh JM. 2013a. Characterization of a novel α-conotoxin TxID from Conus textile that potently blocks rat α3β4 nicotinic acetylcholine receptors. Journal of Medicinal Chemistry 56: 9655-9663. *Chang et al. 2014* : Discovery of a potent and selective α3β4 nicotinic acetylcholine receptor antagonist from an α-conotoxin synthetic combinatorial library.Chang YP, Banerjee J, Dowell C, Wu J, Gyanda R, Houghten RA, Toll L, McIntosh JM, Armishaw CJ. J Med Chem. 2014 Apr 24;57(8):3511-21. *Luo S. et al. 1998*: Luo S, Kulak JM, Cartier GE, Jacobsen RB, Yoshikami D, Olivera BM, McIntosh JM. 1998. alpha-conotoxin AuIB selectively blocks alpha3 beta4 nicotinic acetylcholine receptors and nicotine-evoked norepinephrine release. J Neurosci 18: 8571-8579. *Franco et al. 2012*: Franco A, Kompella SN, Akondi KB, Melaun C, Daly NL, Luetje CW, Alewood PF, Craik DJ, Adams DJ, Mari F. 2012. RegIIA: an alpha4/7-conotoxin from the venom of Conus regius that potently blocks alpha3beta4 nAChRs. Biochem Pharmacol 83: 419-426. *Kompella et al*. *2015*: Kompella SN, Hung A, Clark RJ, Mari F, Adams DJ. 2015. Alanine scan of alpha-conotoxin RegIIA reveals a selective alpha3beta4 nicotinic acetylcholine receptor antagonist. J Biol Chem 290: 1039-1048. *McIntosh et al. 2005*: McIntosh JM, Plazas PV, Watkins M, Gomez-Casati ME, Olivera BM, Elgoyhen AB. 2005. A novel alpha-conotoxin, PeIA, cloned from Conus pergrandis, discriminates between rat alpha9alpha10 and alpha7 nicotinic cholinergic receptors. J Biol Chem 280: 30107-30112. *Dowell et al. 2003*: Dowell C, Olivera BM, Garrett JE, Staheli ST, Watkins M, Kuryatov A, Yoshikami D, Lindstrom JM, McIntosh JM. 2003. Alpha-conotoxin PIA is selective for alpha6 subunit-containing nicotinic acetylcholine receptors. J Neurosci 23: 8445-8452. *Azam et al. 2005:* Azam L, Dowell C, Watkins M, Stitzel JA, Olivera BM, McIntosh JM. 2005. Alpha-conotoxin BuIA, a novel peptide from Conus bullatus, distinguishes among neuronal nicotinic acetylcholine receptors. J Biol Chem 280: 80-87. | | | | | | | | |

It can be seen from Table 2 and Table 3:
Most of the new mutants of α-conotoxin TxID retained the blocking activity against two similar subtypes of α3β4 and α6β4* nAChRs (Table 2), and the discrimination degrees between the two subtypes were mostly within 10 times. The discrimination of wild-type TxID between the two similar subtypes of α3β4 and α6β4* nAChRs was only 9.3 times (Table 3).

However, a good discrimination between the two subtypes α3β4 and α6β4* nAChRs was observed in five new TxID mutants, including SEQ ID NO: 7 that was TxID[S9A], SEQ ID NO: 19 that was TxID[S9R], SEQ ID NO: 24 that was TxID[S9K], SEQ ID NO: 29 that was TxID[14D], and SEQ ID NO: 31 that was TxID[S9E] as shown in Table 2-3. Among them, the polypeptides of SEQ ID NO: 7, 19 and 29 had a high degree of discrimination between the two similar subtypes of α3β4 and α6β4* nAChRs, the discrimination degrees of the three polypeptides were relatively close, and the discrimination degrees were in range of 45-50 times.

It is particularly noteworthy that the novel mutant SEQ ID NO: 24, TxID[S9K], was found to have the best discrimination between the two subtypes, and was a ligand substance so far found with the highest discrimination degree between the two similarly subtypes of α3β4 and α6β4* nAChRs. TxID[S9K] was highly active against α3β4 nAChR with a half-blocking dose of only 10.13 nM, while the blocking activity on α6β4* nAChR was lost, its blocking current was less than 50% at high concentration of 10 µM, and its half-blocking dose was > 10000 nM (Table 2, Table 3, Figure 11C). SEQ ID NO: 31, that is, TxID [S9E], had relatively weak activity against α3β4 nAChR, and its half-blocking dose was 307.61 nM, but it lost blocking activity to α6β4* nAChR, and it had no blocking activity on α6β4* nAChR at high concentration of 10 µM (Fig. 11B, Table 2, Table 3), this mutant peptide showed well discrimination between the two similar subtypes of α3β4 and α6β4* nAChRs.

However, all of the conotoxins found in the prior art have a discrimination degree of less than 20 times for the two subtypes (Table 3); for example, RegIIA, RegIIA [N11, 12A], PeIA, PIA, BuIA show a discrimination degree of 3.1-17.3 times for the two subtypes.

In addition, the present inventors also analyzed in detail the current blocking effect of wild-type TxID, mutant TxID[S9A] and mutant TxID[S9K] at different concentrations on the two subtypes of α3β4 and α6β4* nAChRs, respectively. The results are shown in Figures 12-14. The specific experimental steps refer to the Examples 2-3 above.

Figure 12 and Figure 13 show the comparison of current blocking effect between wild-type TxID and mutant TxID[S9A] on the two subtypes of α3β4 and α6β4* nAChRs, respectively (Figures 12A-12H), and the concentration-dose response curves (Figures 13A-13B).

TxID at 100 nM or 10 nM showed similar current blocking strength on the two subtypes (Figures 12A, 12B, 12E, 12F). TxID at 100 nM blocked the two subtypes of α3β4 and α6β4* nAChRs in current by 97% and 85%, respectively (Figures 12A-12B). TxID at 10 nM blocked the two subtypes of α3β4 and α6β4* nAChRs in current by 67% and 46%, respectively (Figures 12E-12F).

TxID[S9A] at 100 nM or 10 nM showed very different effects on the current blocking for the two receptor subtypes and could distinguish them (Figures 12C, 12D, 12G, 12H). TxID[S9A] at 100 nM blocked the two subtypes α3β4 and α6β4* nAChRs in current by 98% and 26%, respectively (Figures 12C-12D), showing significant difference. TxID[S9A] at 10 nM blocked the current of the α3β4 nAChRs subtype by 75% (Figure 12G), and TxID[S9A] at 10 nM completely lost the blocking activity to the α6β4* nAChRs subtype (Figure 12H), and its current was the same of the control current.

The concentration dose response curves of Figures 13A-13B also reflect that TxID[S9A] had a good discrimination for the two subtypes of α3β4 and α6β4* nAChRs.

TxID[S9K] at 1 µM completely blocked the current of α3β4 nAChR (Figures 14A-14B), while TxID[S9K] with a high concentration of 10 µM showed extremely weak blocking activity on α6/α3β4 nAChR, i.e., less than 1/4 of the control current (Figures 14A-14B).

### Example 5: Spatial structure analysis of TxID and TxID[S9A] by nuclear magnetic resonance (NMR)

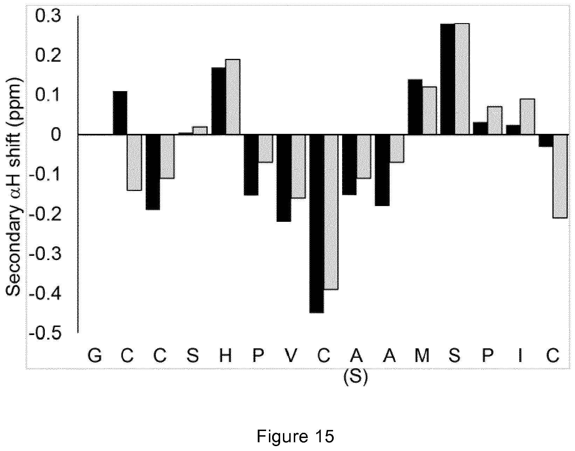

The inventors further analyzed the spatial structure of TxID and TxID[S9A] by nuclear magnetic resonance (NMR), and the specific method referred to Luo S, Zhangsun D, Zhu X, Wu Y, Hu Y, Christensen S, Harvey PJ, Akcan M, Craik DJ, McIntosh JM. 2013a. Characterization of a novel α-conotoxin TxID from Conus textile that potently blocks rat α3β4 nicotinic acetylcholine receptors. Journal of Medicinal Chemistry 56: 9655-9663. Figure 15 showed the analysis results of the secondary chemical shift (ordinate) of TxID[S9A] (isomer 1) and TxID (isomer 1).

NMR structural analysis showed that TxID[S9A] had two conformers in aqueous solution, called isomer 1 (trans-trans isomer, trans isomer) and isomer 2 (cis-trans isomer). At 308 K, the ratio of the two spatial isomers was 70:30. These spatial isomers were formed by the cis-trans isomerization of the peptide bonds preceding the prolines (Pro, P) at the 6th and 13th positions. If the secondary aH chemical shift is 0.1 ppm greater than the random curl value, it indicates that the polypeptide has typical structural features, i.e., the positive shift represents β-type folding structure, and the negative shift represents the α-helical structure (Wishart DS, Bigam CG). , Holm A, Hodges RS, Sykes BD. 1995. 1H, 13C and 15N random coil NMR chemical shifts of the common amino acids. 1. Investigations of nearest-neighbor effects. Journal of Biomolecular NMR 5: 67-81). Figure 15 shows that the trans isomer of TxID[S9A] (isomer 1) has no significant difference in secondary structure characteristics compared to the trans isomer of TxID. Mutation of serine (Ser-9) at position 9 of TxID into alanine (Ala-9) enhanced the α-helical structure of the middle portion of the polypeptide. For cis and trans isomers, both TxID and TxID[S9A] tended to adopt a random coil structure (data were not shown).

Referring to the method of Yu et al. 2012 (Yu, R., Kaas, Q., and Craik, DJ (2012). Delineation of the unbinding pathway of alpha-conotoxin Iml from the alpha7 nicotinic acetylcholine receptor. The Journal of Physical Chemistry B 116 , 6097-6105), a molecular docking analysis of the interaction of α-conotoxin TxID with the ligand binding domain of rat α3β4 or α6β4 nAChRs was performed. The original homology model was constructed using Modeller9v13 (Sali and Blundell 1993) software and two molecular templates. These two molecular templates were the crystal structures of acetylcholine binding protein (AChBP) and α-conotoxin TxIA variant (PDB identifier 2uz6), respectively (Dutertre, S., Ulens, C., Buttner, R., Fish, A., van Elk, R., Kendel, Y., Hopping, G., Alewood, PF, Schroeder, C., Nicke, A., et al. (2007). AChBP-targeted alpha-conotoxin correlates distinct binding orientations With nAChR subtype selectivity. The EMBO Journal 26, 3858-3867), and the crystal structure of the isolated human a9 subunit (PDB identifier 4d01), (Zouridakis et al. 2014). It was assumed that α3β4 and α6β4 nAChRs were pentamers composed of two α-subunits and three β-subunits, and the binding site of conotoxin to the receptor was the interface formed between the a (right side) and β (complementary side) subunits. By using molecular dynamics (MD) simulation software and method with amber ff99SB-ILDN (Lindorff-Larsen, K., Piana, S., Palmo, K., Maragakis, P., Klepeis, J.L., Dror, R.O., and Shaw, D.E. (2010). Improved side-chain torsion potentials for the Amber ff99SB protein force field. Proteins 78, 1950-1958) molecular force field and Gromacs 5.1 MD engine (Pronk, S., Pall, S., Sc Hulz, R., Larsson, P., Bjelkmar, P., Apostolov, R., Shirts, M.R., Smith, J.C., Kasson, P.M., van der Spoel, D., et al. (2013). GROMACS 4.5: a high-throughput and highly parallel open source molecular simulation toolkit. Bioinformatics 29, 845-854), the molecular docking binding model for the interaction of TxID with rat α3β4 or α6β4 nAChRs was refined respectively, and three molecular dynamics (MD) simulation results were obtained (Figure 16).

The molecular docking binding models of TxID to α3β4 and α6β4* nAChRs (Figures 16A-16B) show that although the overall difference between the two was very small, there were still differences, mainly due to the different shape of the binding site, and caused by difference in the amino acid species near the binding site on the a3 and a6 subunits. Molecular dynamics (MD) simulations performed within 50 ns showed that at the binding site to α6β4* nAChRs, a weak hydrogen bond was formed between Ser-9 of TxID and β4 Lys-81 (Figure 16B), while this hydrogen bond was absent at the binding site to α3β4 nAChRs (Figure 16A).

The time-dependent distance between the Ser-9 side chain hydroxyl group of TxID and the β4 Lys-81 side chain nitrogen atom was shown in Figure 16C. Substitution of Ser-9 with alanine disrupted this hydrogen bonding interaction. The results showed that the blocking activity of TxID[S9A] on α6β4* nAChRs was reduced by 5 times compared with that of TxID, which might be attributed to the fact that at 300 K, this point mutation caused an energy decrease of about 1 kcal/mol, which was consistent with the energy of losing a hydrogen bond (Bowie JU. 2011. Membrane protein folding: how important are hydrogen bonds? Curr Opin Struct Biol 21: 42-49). In contrast, at the binding site to α3β4 nAChRs, there was no interaction between Ser-9 of TxID and the receptor, and thus the substitution of S9A had no effect on the blocking activity of α3β4 nAChRs, which was consistent with the experimental results (Table 2-3, Figure 16A).

When the glycine (Gly-1) at position 1 of TxID was substituted with alanine (G1A), the blocking activities against α3β4 and α6/α3β4 nAChRs were decreased by 17 times and 8 times, respectively. According to the molecular docking binding model of Figure 16, there might be a charge interaction between the negatively charged Asp-192 on the β4 subunit and the glycine at position 1 of TxID. This interaction could be attenuated by changes in the bone conformation of TxID [G1A] compared to the wild-type α-conotoxin TxID. The methionine (Met-11) at position 11 of TxID was replaced by isoleucine (Ile) (M11I), resulting in a 20-fold decrease in the blocking activity against α3β4 nAChRs, but there was not a decrease in the blocking activity against α6/α3β4 nAChRs. According to the molecular docking model, Met-11 was in contact with Cys-218 on the C-loop of a3 subunit, and the substitution of M11A might cause a change in binding mode, because a larger side chain would cause a steric hindrance. The M11A mutations resulted in a total loss of blocking activity to both α3β4 and α6/α3β4 nAChRs, and their half-blocking doses were greater than 10000 nM (Table 2). In contrast, in the binding model to α6β4, Met-11 could be substituted by Ile without causing steric hindrance to the binding site on the a6 subunit, thus not affecting the blocking activity of wild-type TxID and point mutation TxID[M11I] against α6/α3β4 nAChRs (Figures 16A-16B, Table 2).

### Example 6: Re-assay of the blocking activities of some new mutants of α-conotoxin TxID on two subtypes of α3β4 and α6/α3β4 nAChRs

The blocking activities of some new mutants of TxID against rat α3β4 and α6/α3β4 nAChRs was measured again by the electrophysiological method in Example 2 using the newly purchased Xenopus oocytes. The results are shown in Table 4. The results showed that the blocking activities of these mutants were basically the same as those of Examples 2 and 3, except that their half-blocking doses (IC₅₀) had slight changes, which were within the range of normal error variation. These results further confirmed the blocking activities of the above mutants on α3β4 and α6/α3β4 nAChRs.

**Table 4. Blocking activities of some new mutants of α-conotoxin TxID on α3β4 and α6/α3β4 nAChRs (half-blocking dose, IC₅₀)**

| SEQ ID NO: | Polypeptide name ^{a} | α3β4 IC₅₀, nM^{c} | α6/α3β4 IC₅₀. nM^{c} |
|---|---|---|---|
| 17 | TxID[S9Abu] | 1.94 (1.53-2.46) | 6.24 (4.64-8.39) |
| 18 | TxID[S9H] | 1.96 (1.45-2.28) | 18.66 (13.43-25.94) |
| 19 | TxID[S9R] | 5.38 (3.98-7.28) | 350 (232.6-526.6) |
| 20 | TxID[S9Y] | 7.93 (6.29-10) | 22.86 (16.24-32.19) |
| 21 | TxID[S9T] | 7.36 (5.91-9.16) | 49.64 (40.61-60.68) |
| 24 | TxID[S9K] | 6.86 (5.27-8.94) | > 10,000 *^{b}* |
| 25 | TxID[S9L] | 9.89 (8.07-12.13) | 20.56 (14.41-29.33) |
| 26 | TxID[S9F] | 10.7 (8.41-13.62) | 10.22 (8.53-12.24) |
| 28 | TxID[S9(D-Arg)] | 52.48 (38.89-70.82) | 430.7 (318.1-583.2) |
| 30 | TxID[S9(D-Ser)] | 124.9 (90.05-173.16) | 502.98 (361.19-700.77) |
| 31 | TxID[S9E] | 319.1 (239.9-424.5) | > 10,000 *^{b}* |
| 32 | TxID[S9D] | 375.6 (239.4-589.5) | > 10,000 *^{b}* |

| | | | |
|---|---|---|---|
| ^{a} Cysteine mode is C₁C₂-C₃-C₄, and disulfide bond linkage mode is 1-3, 2-4. ^{b} Blocking current is less than 50% at a high concentration of 10 µM. ^{c} The number in parentheses indicates the range of half-blocking dose (IC₅₀) for 95% confidence interval. | | | |

Although specific embodiments of the invention have been described in detail, those skilled in the art will understand that various modifications and alterations of the details are possible in light of the teachings of the invention. The full scope of the invention is given by the appended claims and any equivalents thereof.

## Claims

1. An isolated polypeptide having an amino acid sequence as shown in SEQ ID NO: 1 in which the serine at position 9 is substituted by a different L-form or D-form amino acid; preferably, the serine at position 9 in the sequence as shown in SEQ ID NO: 1 is substituted with alanine, 2-aminobutyric acid, histidine, arginine, tyrosine, threonine, lysine, leucine, phenylalanine, D- arginine, D-serine, glutamic acid or aspartic acid.

2. An isolated polypeptide having an amino acid sequence as shown in any one of SEQ ID NOs: 2-3, 7, 9, 11-33, respectively.

3. The polypeptide according to claim 1 or 2, wherein counted from the N-terminus of the polypeptide:
the first cysteine forms a disulfide bond with the third cysteine, and the second cysteine forms a disulfide bond with the fourth cysteine; or the first cysteine forms a disulfide bond with the fourth cysteine, and the second cysteine forms a disulfide bond with the third cysteine; or the first cysteine forms a disulfide bond with the second cysteine, and the third cysteine forms a disulfide bond with the forth cysteine;
preferably, the carboxy terminus of the polypeptide is amidated.

4. An isolated fusion protein comprising at least one polypeptide according to any one of claims 1 to 3.

5. An isolated polynucleotide encoding the polypeptide according to any one of claims 1 to 3.

6. A nucleic acid construct comprising the polynucleotide according to claim 5; preferably, the nucleic acid construct is a recombinant vector; preferably, the nucleic acid construct is a recombinant expression vector.

7. A transformed cell comprising the polynucleotide according to claim 5 or the nucleic acid construct according to claim 6.

8. A pharmaceutical composition comprising at least one polypeptide according to any one of claims 1 to 3; optionally, further comprising a pharmaceutically acceptable excipient.

9. Use of the polypeptide according to any one of claims 1 to 3 in the manufacture of a medicament for blocking an acetylcholine receptor; preferably, the acetylcholine receptor is an α3β4 acetylcholine receptor, or an α6β4* acetylcholine receptor, for example α6/α3β4 acetylcholine receptor.

10. Use of the polypeptide according to any one of claims 1 to 3 in the manufacture of a medicament for the treatment and/or prevention of a nervous system disease or cancer, or in the manufacture of a medicament for killing pests, analgesia, smoking cessation, detoxification or promoting appetite;
preferably, the nervous system disease is at least one of addiction, neuralgia, Parkinson's disease, dementia, schizophrenia and depression;
preferably, the addiction is caused by at least one of the following factors: various psychoactive substances such as nicotine, opium, heroin, methamphetamine (ice), morphine, marijuana, cocaine or alcohol;
preferably, the neuralgia is selected from at least one of the following: sciatica, trigeminal neuralgia, lymphatic neuralgia, multi-point motor neuralgia, acute strenuous spontaneous neuralgia, crush neuralgia, and compound neuralgia;
preferably, the neuralgia is caused by at least one of the following factors: cancer, cancer chemotherapy, alcoholism, diabetes, sclerosis, herpes zoster, mechanical injury, surgical injury, AIDS, head neuralgia, drug poisoning, Industrial pollution poisoning, myeloma, chronic congenital sensory neuropathy, angiitis, vasculitis, ischemia, uremia, childhood bile liver disease, chronic respiratory disorder, multiple organ failure, sepsis/pyaemia, hepatitis, Porphyria, vitamin deficiency, chronic liver disease, native biliary sclerosis, hyperlipidemia, leprosy, Lyme arthritis, sensory perineuritis or allergies;
preferably, the cancer is a lung cancer such as small cell lung cancer, ovarian cancer, leukemia, neuroblastoma or breast cancer.

11. A method of blocking an acetylcholine receptor or modulating a acetylcholine level in vivo or in vitro, comprising a step of administering to a subject or administering to a cell an effective amount of the polypeptide according to any one of claims 1 to 3; preferably, the acetylcholine receptor is an α3β4 acetylcholine receptor, or an α6β4* acetylcholine receptor such as an α6/α3β4 acetylcholine receptor.

12. The polypeptide according to any one of claims 1 to 3, for use in blocking an acetylcholine receptor; preferably, the acetylcholine receptor is an α3β4 acetylcholine receptor, or an α6β4* acetylcholine receptor such as α6/α3β4 acetylcholine receptor.

13. The polypeptide according to any one of claims 1 to 3, for use in the treatment and/or prevention of a nervous system disease or cancer, or for use in killing pests, analgesia, smoking cessation, detoxification or promoting appetite;
preferably, the nervous system disease is at least one of addiction, neuralgia, Parkinson's disease, dementia, schizophrenia and depression;
preferably, the addiction is caused by at least one of the following factors: various psychoactive substances such as nicotine, opium, heroin, methamphetamine (ice), morphine, marijuana, cocaine or alcohol;
preferably, the neuralgia is selected from at least one of the following: sciatica, trigeminal neuralgia, lymphatic neuralgia, multi-point motor neuralgia, acute strenuous spontaneous neuralgia, crush neuralgia, and compound neuralgia;
preferably, the neuralgia is caused by at least one of the following factors: cancer, cancer chemotherapy, alcoholism, diabetes, sclerosis, herpes zoster, mechanical injury, surgical injury, AIDS, head neuralgia, drug poisoning, Industrial pollution poisoning, myeloma, chronic congenital sensory neuropathy, angiitis, vasculitis, ischemia, uremia, childhood bile liver disease, chronic respiratory disorder, multiple organ failure, sepsis/pyaemia, hepatitis, Porphyria, vitamin deficiency, chronic liver disease, native biliary sclerosis, hyperlipidemia, leprosy, Lyme arthritis, sensory perineuritis or allergies;
Preferably, the cancer is a lung cancer such as small cell lung cancer, ovarian cancer, leukemia, neuroblastoma or breast cancer.

14. A method for treating and/or preventing a nervous system disease or cancer, or a method for killing pests, analgesia, smoking cessation, detoxification or promoting appetite, comprising a step of administering to a subject in need thereof an effective amount of the polypeptide according to any one of claims 1 to 3;
preferably, the nervous system disease is at least one of addiction, neuralgia, Parkinson's disease, dementia, schizophrenia and depression;
preferably, the addiction is caused by at least one of the following factors: various psychoactive substances such as nicotine, opium, heroin, methamphetamine (ice), morphine, marijuana, cocaine or alcohol;
preferably, the neuralgia is selected from at least one of the following: sciatica, trigeminal neuralgia, lymphatic neuralgia, multi-point motor neuralgia, acute strenuous spontaneous neuralgia, crush neuralgia, and compound neuralgia;
preferably, the neuralgia is caused by at least one of the following factors: cancer, cancer chemotherapy, alcoholism, diabetes, sclerosis, herpes zoster, mechanical injury, surgical injury, AIDS, head neuralgia, drug poisoning, Industrial pollution poisoning, myeloma, chronic congenital sensory neuropathy, angiitis, vasculitis, ischemia, uremia, childhood bile liver disease, chronic respiratory disorder, multiple organ failure, sepsis/pyaemia, hepatitis, Porphyria, vitamin deficiency, chronic liver disease, native biliary sclerosis, hyperlipidemia, leprosy, Lyme arthritis, sensory perineuritis or allergies;
Preferably, the cancer is a lung cancer such as small cell lung cancer, ovarian cancer, leukemia, neuroblastoma or breast cancer.
